(19)

**Europäisches Patentamt**
**European Patent Office**
**Office européen des brevets**

(11) **EP 1 659 184 B1**

(12) **EUROPÄISCHE PATENTSCHRIFT**

(45) Veröffentlichungstag und Bekanntmachung des Hinweises auf die Patenterteilung:
**07.07.2010 Patentblatt 2010/27**

(51) Int Cl.:
***C12Q 1/68*** (2006.01)

(21) Anmeldenummer: **04105933.8**

(22) Anmeldetag: **19.11.2004**

(54) **Genetischer Test auf Strabismus beim Rind**

Genetic test for strabism in cattle

Test genétique pour détecter le strabisme chez le bétail

(84) Benannte Vertragsstaaten:
**AT BE BG CH CY CZ DE DK EE ES FI FR GB GR HU IE IS IT LI LU MC NL PL PT RO SE SI SK TR**

(43) Veröffentlichungstag der Anmeldung:
**24.05.2006 Patentblatt 2006/21**

(73) Patentinhaber: **Stiftung Tierärztliche Hochschule Hannover**
**30559 Hannover (DE)**

(72) Erfinder:
• **Distl, Ottmar**
  **30559 Hannover (DE)**
• **Drögemüller, Cord**
  **30169 Hannover (DE)**
• **Mömke, Stefanie**
  **30559 Hannover (DE)**
• **Wöhlke, Anne**
  **30539 Hannover (DE)**

(74) Vertreter: **Taruttis, Stefan Georg et al**
**TARUTTIS Patentanwaltskanzlei**
**Aegidientorplatz 2b**
**30159 Hannover (DE)**

(56) Entgegenhaltungen:
**WO-A-02/14544     WO-A-92/13102**

• **BISHOP M D ET AL: "A GENETIC LINKAGE MAP FOR CATTLE" GENETICS, GENETICS SOCIETY OF AMERICA, AUSTIN, TX, US, Bd. 136, Nr. 2, 1994, Seiten 619-639, XP002058402 ISSN: 0016-6731**

• **KOSSAREK L M ET AL: "SIX BOVINE DINUCLEOTIDE REPEAT POLYMORPHISMS: RM041, RM051, RM066, RM088, RM103 AND RM113" ANIMAL GENETICS, BLACKWELL SCIENTIFIC PUBLICATIONS, LONDON, GB, Bd. 26, Nr. 1, 1995, Seiten 55-56, XP009048851 ISSN: 0268-9146**

• **STONE R T ET AL: "A SMALL-INSERT BOVINE GENOMIC LIBRARY HIGHLY ENRICHED FOR MICROSATELLITE REPEAT SEQUENCES" MAMMALIAN GENOME, NEW YORK, NY, US, Bd. 6, Nr. 10, 1995, Seiten 714-724, XP000982207 ISSN: 0938-8990**

• **DATABASE GENBANK 27. August 2004 (2004-08-27), "Bos taurus keratin 5 PCR primer sequence SEQ.ID.24" XP002334051 Database accession no. ADW07184**

• **DATABASE GEN 27. August 2004 (2004-08-27), "Bos taurus keratin 5 PCR primer sequence SEQ.ID.NO.25" XP002334052 Database accession no. ADW07185**

• **GERST M ET AL: "VERBREITUNG UND GENETIK DES BILATERALEN STRABISMUS CONVERGENS MIT EXOPHTHALMUS BEIM RIND OCCURRENCE AND GENETICS OF BILATERAL CONVERGENT STRABISMUS WITH EXOPHTHALMUS IN CATTLE" TIERAERZTLICHE UMSCHAU, KONSTANZ, DE, Bd. 53, Nr. 1, 1. Januar 1998 (1998-01-01), Seiten 6-8,11, XP009047579 ISSN: 0049-3864**

• **DISTL O ET AL: "Association analysis between bilateral convergent strabismus with exophthalmus and milk production traits in dairy cattle" JOURNAL OF VETERINARY MEDICINE SERIES A, Bd. 47, Nr. 1, Februar 2000 (2000-02), Seiten 31-36, XP002334031 ISSN: 0931-184X**

EP 1 659 184 B1

- GERST M ET AL: "EINFLUESSE AUF DIE DISSEMINATION DES BILATERALEN STRABISMUS CONVERGENS MIT EXOPHTHALMUS BEIM RIND INFLUENCES ON THE DISSEMINATION OF BILATERAL CONVERGENT STRABISMUS WITH EXOPHTHALMUS IN DAIRY CATTLE" ARCHIV FUER TIERZUCHT, FORSCHUNGSINSTITUT FUER DIE BIOLOGIE LANDWIRTSCHAFTLICHER, DE, Bd. 40, Nr. 5, 1997, Seiten 401-412, XP009047580 ISSN: 0003-9438
- FUJIWARA HIROTAKE ET AL: "Genome-wide search for strabismus susceptibility loci." ACTA MEDICA OKAYAMA, Bd. 57, Nr. 3, Juni 2003 (2003-06), Seiten 109-116, XP002334032 ISSN: 0386-300X
- PARIKH VAISHALI ET AL: "A strabismus susceptibility locus on chromosome 7p." PROCEEDINGS OF THE NATIONAL ACADEMY OF SCIENCES OF THE UNITED STATES OF AMERICA, Bd. 100, Nr. 21, 14. Oktober 2003 (2003-10-14), Seiten 12283-12288, XP002334033 ISSN: 0027-8424
- FRIES R ET AL: "THE BOVINE GENOME CONTAINS POLYMORPHIC MICROSATELLITES" GENOMICS, ACADEMIC PRESS, SAN DIEGO, US, Bd. 8, Nr. 2, Oktober 1990 (1990-10), Seiten 403-406, XP000406207 ISSN: 0888-7543
- KEMP S J ET AL: "A PANEL OF BOVINE, OVINE AND CAPRINE POLYMORPHIC MICROSATELLITES" ANIMAL GENETICS, BLACKWELL SCIENTIFIC PUBLICATIONS, LONDON, GB, Bd. 24, Nr. 5, Oktober 1993 (1993-10), Seiten 363-365, XP000990035 ISSN: 0268-9146
- MA R Z ET AL: "ISOLATION AND CHARACTERIZATION OF 45 POLYMORPHIC MICROSATELLITES FROM THE BOVINE GENOME" ANIMAL GENETICS, BLACKWELL SCIENTIFIC PUBLICATIONS, LONDON, GB, Bd. 27, Nr. 1, 1996, Seiten 43-47, XP009048888 ISSN: 0268-9146
- REED K M ET AL: "Development of 90 new bovine microsatellite loci" ANIMAL BIOTECHNOLOGY, Bd. 12, Nr. 1, Mai 2001 (2001-05), Seiten 69-76, XP009048847 ISSN: 1049-5398
- SONSTEGARD T S ET AL: "AN INTEGRATED GENETIC AND PHYSICAL MAP OF THE BOVINE X CHROMOSOME" MAMMALIAN GENOME, NEW YORK, NY, US, Bd. 8, Nr. 1, 1997, Seiten 16-20, XP000982206 ISSN: 0938-8990
- KAPPES ET AL: "A second-generation linkage map of the bovine genome" GENOME RESEARCH, COLD SPRING HARBOR LABORATORY PRESS, US, Bd. 7, 1997, Seiten 235-249, XP002085806 ISSN: 1088-9051
- NAPOLITANO F ET AL: "EXPLOITATION OF MICROSATELLITES AS GENETIC MARKERS OF BEEF- PERFORMANCE TRAITS IN PIEMONTESEXCHIANINA CROSSBRED CATTLE" JOURNAL OF ANIMAL BREEDING AND GENETICS, BLACKWELL WISSENSCHAFTS VERLAG, BERLIN, DE, Bd. 113, Nr. 3, 1. Juni 1996 (1996-06-01), Seiten 157-162, XP000197181 ISSN: 0931-2668
- YAMADA KOKI ET AL: "Heterozygous mutations of the kinesin KIF21A in congenital fibrosis of the extraocular muscles type 1 (CFEOM1)." NATURE GENETICS, Bd. 35, Nr. 4, Dezember 2003 (2003-12), Seiten 318-321, XP002334049 ISSN: 1061-4036
- YAMADA KOKI ET AL: "Identification of KIF21A mutations as a rare cause of congenital fibrosis of the extraocular muscles type 3 (CFEOM3)." INVESTIGATIVE OPHTHALMOLOGY & VISUAL SCIENCE. JUL 2004, Bd. 45, Nr. 7, Juli 2004 (2004-07), Seiten 2218-2223, XP009048869 ISSN: 0146-0404
- TAUTZ D: "HYPERVARIABILITY OF SIMPLE SEQUENCES AS GENERAL SOURCE FOR POLYMORPHIC DNA MARKERS" NUCLEIC ACIDS RESEARCH, OXFORD UNIVERSITY PRESS, SURREY, GB, Bd. 17, Nr. 16, 1989, Seiten 6463-6471, XP002161967 ISSN: 0305-1048
- PATERSON A H ET AL: "RESOLUTION OF QUANTITATIVE TRAITS INTO MENDELIAN FACTORS BY USING A COMPLETE LINKAGE MAP OF RESTRICTION FRAGMENT LENGHT POLYMORPHISMS" NATURE, MACMILLAN JOURNALS LTD. LONDON, GB, Bd. 335, 1988, Seiten 721-726, XP000910123 ISSN: 0028-0836
- ABECASIS GONCALO R ET AL: "Merlin: Rapid analysis of dense genetic maps using sparse gene flow trees" NATURE GENETICS, Bd. 30, Nr. 1, Januar 2002 (2002-01), Seiten 97-101, XP002334050 ISSN: 1061-4036

**Beschreibung**

Die vorliegende Erfindung bezieht sich auf einen genetischen Test von Rindern, mit dem festgestellt werden kann, ob ein Tier Träger der Erbanlagen ist, die zum beidseitigen konvergierenden Schielen (Strabismus) mit Hervortreten der Augäpfel aus der Augenhöhle (Exophthalmus) führt, auch als BCSE bezeichnet.

Als Ergebnis komplexer Segregationsanalysen konnte festgestellt werden, dass für den Strabismus bei Rindern ein autosomal dominantes Hauptgen ursächlich ist. Bisher konnte BCSE bei Rindern der Rassen Jersey, British Shorthorn, Deutsche Schwarzbunte, Deutsches Braunvieh, Deutsches Fleckvieh und Irish Friesian beobachtet werden.

Bei der Überprüfung der Herkunft des verantwortlichen Defektallels und dessen Verbreitung beim Deutschen Braunvieh zeigte sich, dass das Defektallel vorwiegend innerhalb Kuhfamilien und Herden segregiert und eine Verbreitung über Besamungsbullen nur im begrenzten Umfang möglich erscheint. Eine Übertragung des Defektallels über den Pfad von der Bullenmutter zum Besamungsbullen würde zu einem massiven Anstieg der Befallshäufigkeit führen. Daher ist die sorgfältige Überprüfung der Bullenmutterfamilien und Prüfbullen äußerst vordringlich, um eine Verbreitung dieses Defektallels zu vermeiden.

Die WO 02/14544 beschreibt ein Verfahren zur Bestimmung genetischer Eigenschaften von Rinderembryonen, wobei Mikrosatelliten als Marker eingesetzt werden. Dabei wird die Abstammung der Tiere geprüft oder das Geschlecht und die Fellfarbe an einzelnen Zellen identifiziert.

Die WO 92/13102 beschreibt eine Vielzahl von Mikrosatelliten, die als Marker zur Analyse des Stammbaums von Rindern eingesetzt werden.

Bishop et al., Genetics, 136, 619-639 (1994) beschreiben die Verwendung einer Vielzahl von Mikrosatelliten als genetische Marker für eine Karte des Rindergenoms in Form von Kopplungsgruppen, die den Chromosomen zugeordnet werden.

Kossarek et al., Animal Genetics, 26, S. 55 beschreiben Polymorphismen von Mikrosatelliten, die in Rinderfamilien vererbt werden.

Stone et al., Mammalian Genome, 6, 714-724 (1995) beschreiben die Verwendung von Mikrosatelliten als Marker für eine Genbibliothek in Phagen.

Im Stand der Technik werden zwar Mikrosatelliten genannt, jedoch wird die Verwendung von Mikrosatelliten zur Identifikation von Defektallelen, die für BCSE ursächlich sind, nicht angesprochen oder angeregt.

Bisher konnte das Defektallel nicht identifiziert werden und auch der Versuch, ein homologes Gen für das beim Menschen für den Strabismus verantwortliche Gen auch beim Rind aufzufinden, war nicht erfolgreich.

Es ist schwierig, die Diagnose auf Strabismus für ein Rind zu stellen, da der Phänotyp meist erst ab einem Alter von 6 bis 24 Monaten oder noch später im Leben auftritt. Eine Altersgrenze, ab der BCSE nicht mehr auftritt, kann jedoch nicht angegeben werden.

Der Augendefekt BCSE ist tierschutzrelevant, sodass sich der vorliegenden Erfindung die Aufgabe stellt, einen verlässlichen Test für die Analyse dafür bereitzustellen, ob ein Tier Träger der für BCSE verantwortlichen Erbanlagen ist. Denn Tiere mit einem solchen genetischen Defekt sind von der Zucht auszuschließen.

**Allgemeine Beschreibung der Erfindung**

Die Erfinder konnten zeigen, dass bestimmte Mikrosatelliten eng mit den Genorten gekoppelt sind, deren Defektallele Ursache für BCSE sind. Die Kopplung dieser Mikrosatelliten mit den verantwortlichen Genorten ist sehr eng, sodass die Mikrosatelliten, bei den bisherigen Analysen immer mit den entsprechenden Defektallelen gekoppelt waren, d.h. während der Vererbung trat eine Trennung der Defektallele von den gekoppelten Mikrosatelliten nicht oder im wesentlichen nicht auf.

Die Mikrosatelliten sind auch deshalb als Marker für die Genorte geeignet, die zu BCSE führen, da sie hoch polymorph sind. Auf diese Weise ist es möglich, eine eindeutige Zuordnung der identifizierten Länge, d.h. der Ausprägung der Mikrosatelliten zu einem bestimmten Defektallel vorzunehmen, das für BCSE bei Rindern verantwortlich ist.

Erfindungsgemäß werden die Ausprägungen, d.h. die Größen der spezifisch zu analysierenden Mikrosatelliten mit dem Phänotyp der Individuen in Beziehung gesetzt, die zu einem Stammbaum gehören. Diese Zuordnung mittels einer Kopplungsanalyse von miteinander verwandten Tieren bekannten Phänotyps führt im Ergebnis zur Identifikation solcher Mikrosatelliten, die innerhalb eines Stammbaums Defektallele für BCSE anzeigen. Die Identifikation der Mikrosatelliten, die innerhalb eines Stammbaums mit dem Phänotyp BCSE gekoppelt sind, ist deshalb erforderlich, um eine verlässliche Aussage darüber zu treffen, welche Ausprägungen der spezifischen Mikrosatelliten mit einem Defektallel gekoppelt sind. Denn die Mikrosatelliten sind nur physikalisch mit dem für BCSE verantwortlichen Genort gekoppelt, sodass die Ausprägung der Mikrosatelliten erst nach Zuordnung zu einem Haplotyp als Marker für gesunde oder defekte Allele dienen kann. Die hochgradige Polymorphie der Mikrosatelliten bringt es mit sich, dass diese jeweils innerhalb eines Stammbaums ein spezifisches Ausprägungsmuster, d.h. eine spezifische Größe entsprechend der Anzahl der jeweiligen Struktureinheiten zeigen und daher individuell identifizierbar sind.

Der Vorteil dieser Mikrosatelliten liegt darin, dass sie derart eng mit dem für BCSE verantwortlichen Genort gekoppelt sind, dass sie im Vererbungsgang an diesen gekoppelt bleiben und andererseits, dass sie hochgradig polymorph sind. Polymorphie bedeutet, dass die mit den für BCSE verantwortlichen Genorten gekoppelten Mikrosatelliten dieselben definierten Struktureinheiten aufweisen und mit denselben Primerpaaren amplifiziert werden können, die Anzahl an Struktureinheiten jedoch von Stammbaum zu Stammbaum abweicht. Auf diese Weise ist für einen Stammbaum eine individuelle Zuordnung der Ausprägung dieser Mikrosatelliten möglich, d.h. der Länge ihrer spezifischen Amplifikate zu bestimmten Allelen, deren Defekt über die Stammbaumanalyse den bekannten Phänotypen zugeordnet werden kann.

Zur Analyse der Mikrosatelliten sind Primerpaare geeignet, die stromauf und stromab von den jeweiligen Mikrosatelliten liegen und für diese spezifisch sind. Die in den Tabellen 1 und 2 angeführten Primerpaare sind zur Identifikation der Mikrosatelliten mit den definierten Struktureinheiten spezifisch, es ist jedoch möglich, andere Primerpaare zu identifizieren, mit denen ebenfalls die Mikrosatelliten identifiziert und per Polymerase-Kettenreaktion (PCR) spezifisch amplifiziert werden können.

**Genaue Beschreibung der Erfindung**

Die vorliegende Erfindung verwendet die Polymerase-Kettenreaktion als Nachweisverfahren für die Mikrosatelliten. Bei der Ausführung der Erfindung reicht es generell aus, mit Primerpaaren, die für jeweils einen Mikrosatelliten spezifisch sind, diesen Bereich zu amplifizieren und mittels der Gel-Elektrophorese die Größe des Amplifikats zu bestimmen. Die Größe des Amplifikats wird durch die Anzahl der jeweils vorhandenen Strukturelemente bestimmt, einschließlich der stromauf und stromab flankierenden Bereiche mit den stromauf bzw. stromab verwendeten Primern. Die in den Tabellen 1 und 2 angegebenen Längen der Amplifikate der PCR enthalten daher neben der für einen Stammbaum spezifischen Anzahl der definierten Struktureinheiten die flankierenden Bereiche des Mikrosatelliten, die einschließlich der Primer-Sequenzen jeweils mit amplifiziert werden.

Erfindungsgemäß werden die Ausprägungen, d.h. die Größen der spezifischen Mikrosatelliten mit dem Phänotyp der Individuen korreliert, die zu einem Stammbaum gehören. Mittels einer Kopplungsanalyse von miteinander verwandten Tieren bekannten Phänotyps können im Ergebnis Mikrosatelliten den gesunden bzw. defekten Allelen des Genorts für BCSE zugeordnet werden. Die Mikrosatelliten sind nicht für BCSE ursächlich, jedoch hinreichend eng mit dem verantwortlichen Allel gekoppelt, sodass die Ausprägung der Mikrosatelliten nach Zuordnung zu einem Haplotyp als Marker für gesunde oder defekte Allele innerhalb einer Familie dienen kann.

Die Mikrosatelliten auf Rinderchromosom Nr. 5 sind mit den Genen KIF21A (Kinesin family member 21A), PRPH (Peripherin), AAAS (Achalasia, adrenocortical insufficiency, alacrimia (Allgrove, triple-A)) gekoppelt, sowie BP1, BL 23, RM103, AGLA293, BMS1315, OarFCB5, BMS321, BMC1009, BMS1898, bzw. sind auf Rinderchromosom Nr. 18 mit den Genen SLC27A5 (Solute carrier family 27 (fatty acid transporter), member 5), KCNJ14 (Potassium inwardly-rectifiying channel, subfamilyJ, member 14) gekoppelt, sowie BMS2785, MS936FBN (=DIK4013), BM2078, BM6507 und TGLA227.

Die bisherigen Analysen zeigen, dass der Phänotyp der BCSE mit hoher Wahrscheinlichkeit nur bei Tieren auftritt, die ein Defektallel sowohl auf Chromosom Nr. 5 als auch Chromosom Nr. 18 zumindest jeweils heterozygot tragen. So tritt der Phänotyp des BCSE mit hoher Wahrscheinlichkeit nicht bei Tieren auf, die ein Defektallel nur für eins der Chromosomen Nr. 5 oder Nr. 18 geerbt haben.

Die Tabelle 1 gibt die Primer-Paare an, mit denen die jeweiligen Mikrosatelliten mittels der PCR spezifisch amplifiziert werden können.

In der bevorzugten Ausführungsform der Erfindung werden die Primer-Paare für 14 der Marker, die in Tabellen 1 und 2 definiert sind, zu insgesamt 8 Reaktionsansätzen der PCR zusammengefasst, sodass nur 5 verschiedene PCR-Ansätze für die Marker von Chromosom Nr. 5 und drei PCR-Ansätze für die Marker von Chromosom Nr. 18 erforderlich sind.

Tabelle 1: Mikrosatelliten auf Rinder-Chromosom Nr. 5

| Marker | Position (cM) | Sequenz der Primer (5'->3') | HT[1] | Amplifik at (bp) | Anzahl möglicher Allele | Struktureinheit |
|---|---|---|---|---|---|---|
| BP1 | 17.29 | Seq.-ID Nr. 1: Vorwärts AAAATCCCTTCATAACAGTGCC<br>Seq.-ID Nr. 2: Rückwärts CATCGTGAATTCCAGGGTTC | 56 | 302-332 | 16 | n.a. |
| DIK28 28 | 20.11 | Seq.-ID Nr. 3: Vorwärts TGGTGTCTGCCAAAGCAGTA<br>Seq.-ID Nr. 4: Rückwärts CGGGAATTTTCAGAGGGAAT | 60 | 228-248 | 10 | Seq.-ID Nr. 57: $(CA)_{17}$ |
| BL23 | 30.13 | Seq.-ID Nr. 5: Vorwärts TCAATCCCTGGGTCAGGAAG<br>Seq.-ID Nr. 6: Rückwärts CTTATTTCAAAATTCGGTTGGG | 54 | 242-256 | 5 | n.a. |
| RM103 | 29.43 | Seq.-ID Nr. 7: Vorwärts<br>TCTGTGCACTTTACATTTAACAGA<br>Seq.-ID Nr. 8: Rückwärts<br>GTGGTCTATTGAACTTTTGTTCAGA | 58 | 114-136 | 12 | Seq.-ID Nr. 58: $(CA)_{20}$ |
| DIK42 98 | 30.14 | Seq.-ID Nr. 9: Vorwärts CCCATGACCCAACAAGCAG<br>Seq.-ID Nr. 10: Rückwärts GCCTCAGAGAAATGGGAACA | 60 | 191-206 | 8 | Seq.-ID Nr. 59: $(TG)_{15}$ |
| DIK27 18 | 30.14 | Seq.-ID Nr. 11: Vorwärts AGGAAGGACAAGGACATTGC<br>Seq.-ID Nr. 12: Rückwärts AGAGGGTCAAAGGCTTAATGG | 55 | 177-195 | 7 | Seq.-ID Nr. 60: $(TG)_{19}$ |
| DIK21 35 | 30.49 | Seq.-ID Nr. 13: Vorwärts ACAATCCATGGGGTAGCAAA<br>Seq.-ID Nr. 14: Rückwärts TTTGGCACTGCAAGAAAGTG | 60 | 220-232 | 6 | Seq.-ID Nr. 61: $(TG)_{12}$ |
| AGLA 293 | 32.25 | Seq.-ID Nr. 15: Vorwärts<br>GAAACTCAACCCAAGACAACTCAAG<br>Seq.-ID Nr. 16: Rückwärts<br>ATGACTTTATTCTCCACCTAGCAGA | 54 | 237-257 | 11 | n.a. |
| BMS13 15 | 33.66 | Seq.-ID Nr. 17: Vorwärts AAGCCATTGATTGTAGATTGGG<br>Seq.-ID Nr. 18: Rückwärts GAGTTTCCTTTTTCCCCCAC | 58 | 133-157 | 11 | Seq.-ID Nr. 62: $(TG)_{19}$ |

EP 1 659 184 B1

| Marker | Position (cM) | Sequenz der Primer (5'->3') | HT[1] | Amplifik at (bp) | Anzahl möglicher Allele | Struktureinheit |
|---|---|---|---|---|---|---|
| DIK50 02 | 33.66 | Seq.-ID Nr. 19: Vorwärts TGTGCTGGAGGTGATAGCTG<br>Seq.-ID Nr. 20: Rückwärts TGCAGGAATATGAGAGCTGAGA | 55 | 202-216 | 7 | Seq.-ID Nr. 63: $(TG)_{10}$ |
| OARF CB5 | 35.28 | Seq.-ID Nr. 21: Vorwärts<br>**GACCTGACCCTTACTCTCTTCAC TC**<br>Seq.-ID Nr. 22: Rückwärts<br>**AAGTTAATTTTCTGGCTGGAAAA CCC** | 56 | 77-93 | 6 | Seq.-ID Nr. 64: $(TG)_{16}$ |
| DIK25 83 | 35.86 | Seq.-ID Nr. 23: Vorwärts CCAGAGGAGGTGGAGAGACA<br>Seq.-ID Nr. 24: Rückwärts CCAAATATCTCAGGGCTGCT | 58 | 186-202 | 6 | Seq.-ID Nr. 65: $(CA)_{17}$ |
| BM321 | 38.24 | Seq.-ID Nr. 25: Vorwärts AAGGGTCAGACAAAACTTAGCA<br>Seq.-ID Nr. 26: Rückwärts ATCCTTGCCCTAATTCTCATTC | 60 | 108-126 | 7 | Seq.-ID Nr. 66: $(CA)_{19}$ |
| DIK47 59 | 40.29 | Seq.-ID Nr. 27: Vorwärts AGTTGGACCTGCCATTGTTC<br>Seq.-ID Nr. 28: Rückwärts ACTTATGTGCGTGCGTGCT | 55 | 212-227 | 8 | Seq.-ID Nr. 67: $(TG)_{19}$ |
| BMC10 09 | 41.69 | Seq.-ID Nr. 29: Vorwärts GCACCAGCAGAGAGGACATT<br>Seq.-ID Nr. 30: Rückwärts ACCGGCTATTGTCCATCTTG | 58 | 256-289 | 13 | n.a. |
| DIK24 65 | 41.69 | Seq.-ID Nr. 31: Vorwärts ATGCACCACAGCGTTCATAG<br>Seq.-ID Nr. 32: Rückwärts TCACGAAGAGAGGCTAACACTG | 55 | 168-182 | 7 | Seq.-ID Nr. 68: $(CA)_{21}$ |
| BMS18 98 | 44.48 | Seq.-ID Nr. 33: Vorwärts GACATCACTTTGGGGCATG<br>Seq.-ID Nr. 34: Rückwärts TCTCCCCACCTCTTCCATC | 58 | 90-112 | 10 | Seq.-ID Nr. 69: $(TG)_{22}$ |
| CSSM3 4 | 45.51 | Seq.-ID Nr. 35: Vorwärts CAGGCCATAACTCTGGGACT<br>Seq.-ID Nr. 36: Rückwärts TCAGCCATCTCTCCTTGTCC | 60 | 93-115 | 10 | Seq.-ID Nr. 70: $(TG)_{25}$ |

EP 1 659 184 B1

(fortgesetzt)

| Marker | Position (cM) | Sequenz der Primer (5'->3') | HT[1] | Amplifik at (bp) | Anzahl möglicher Allele | Struktureinheit |
|---|---|---|---|---|---|---|
| DIK43 52 | 47.51 | Seq.-ID Nr. 37: Vorwärts GTTGTTGCAAATGGCATGAT<br>Seq.-ID Nr. 38: Rückwärts GGCATGGAATTAGGGTTGAA | 58 | 195-211 | 7 | Seq.-ID Nr. 71: $(TG)_{14}$ |
| [1]HT: Hybridisierungstemperatur | | | | | | |

(a): Die Primer für die Mikrosatelliten mit der Kennzeichnung (a), nämlich RM103, BMS1315, BMC1009 und BMS1898 können zur Amplifizierung in einem PCR-Ansatz zusammengefaßt werden, wobei vorzugsweise eine Hybridisierungstemperatur von 58 ˚C und 36 Amplifizierungszyklen verwendet werden.

(b): Die Primer für die Mikrosatelliten mit der Kennzeichnung (b), nämlich BL 23 und AGLA293 können zur Amplifizierung in einem PCR-Ansatz zusammengefaßt werden, wobei vorzugsweise eine Hybridisierungstemperatur von 54 ˚C und 36 Amplifizierungszyklen verwendet werden.

Die Mikrosatelliten BP1, OARFCB5 und BMS321 werden vorzugsweise in Einzelansätzen mit der angegebenen Hybridisierungstemperatur und 36, 36 bzw. 34 Amplifizierungszyklen verwendet.

Tabelle 2: Mikrosatelliten auf Rinder-Chromosom Nr. 18

| Marker | Position (cM) | Sequenz der Primer (5'->3') | HT[1] | Amplifik at (bp) | Anzahl möglicher Allele | Struktureinheit |
|---|---|---|---|---|---|---|
| BMS27 85 | 72.01 | Seq.-ID Nr. 39: Vorwärts ACAAACCTGTGCGCCTTG <br> Seq.-ID Nr. 40: Rückwärts GGCAATCAGTCGGACACAC | 58 | 105-115 | 4 | Seq.-ID Nr. 72: $(TG)_{15}$ |
| DIK26 96 | 76.39 | Seq.-ID Nr. 41: Vorwärts GTGGGGAACAGGTTGTTGAA <br> Seq.-ID Nr. 42: Rückwärts ACCCACTCCACTGTTCTTGC | 58 | 199-205 | 4 | Seq.-ID Nr. 73: $(TG)_{12}$ |
| BM207 8 | 76.78 | Seq.-ID Nr. 43: Vorwärts CCCAAAAGAAGCCAGGAAG <br> Seq.-ID Nr. 44: Rückwärts TCAGAGTTTGGGGTCCTCAG | 56 | 92-114 | 11 | Seq.-ID Nr. 74: $(CA)_{15}$ |
| DIK51 09 | 77.6 | Seq.-ID Nr. 45: Vorwärts GCACAGAGATGCTCGGTCTC <br> Seq.-ID Nr. 46: Rückwärts GCTCAGATCCTCCGCACTTA | 55 | 188-200 | 4 | Seq.-ID Nr. 75: $(TG)_{11}$ |
| DIK49 43 | 77.6 | Seq.-ID Nr. 47: Vorwärts AGACAGGGCCTGATGAATGT <br> Seq.-ID Nr. 48: Rückwärts CCCACCTCTCACAACACACA | 55 | 93-95 | 2 | Seq.-ID Nr. 76: $(TG)_{22}$ |
| BM650 7 | 78.85 | Seq.-ID Nr. 49: Vorwärts ACTTAGCACAATGCCCTCTAGG <br> Seq.-ID Nr. 50: Rückwärts ATGTTATTCCATCAGGAGGAGC | 58 | 146-160 | 8 | Seq.-ID Nr. 77: $(CA)_{15}$ |
| DIK52 35 | 79.47 | Seq.-ID Nr. 51: Vorwärts TCGTGTGCTGTTCATTGTCA <br> Seq.-ID Nr. 52: Vorwärts TCAGCAGGCAGGTCCTTTAT | 55 | 189-203 | 6 | Seq.-ID Nr. 78: $(CA)_{18}$ |
| TGLA2 27 | 84.09 | Seq.-ID Nr. 53: Vorwärts <br><br> CGAATTCCAAATCTGTTAATTTGCT <br><br> Seq.-ID Nr. 54: Rückwärts <br><br> ACAGACAGAAACTCAATGAAAGCA | 56 | 76-102 | 14 | n.a. |
| MS936 FBN = DIK40 13 | 84.38 | Seq.-ID Nr. 55: Vorwärts GAAATTTGTGACCCCTGCAT <br> Seq.-ID Nr. 56: Rückwärts CTAAAGCTCTGCCTCCCAAG | 55 | 165-193 | 12 | Seq.-ID Nr. 79: $(TG)_{25}$ |

EP 1 659 184 B1

## EP 1 659 184 B1

(c): Die Primer für die Mikrosatelliten mit der Kennzeichnung (c), nämlich BM2078 und TGLA227 können zur Amplifizierung in einem PCR-Ansatz zusammengefaßt werden, wobei vorzugsweise eine Hybridisierungstemperatur von 56 °C und 34 Amplifizierungszyklen verwendet werden.

(d): Die Primer für die Mikrosatelliten mit der Kennzeichnung (d), nämlich BMS2785 und BM6507 können zur Amplifizierung in einem PCR-Ansatz zusammengefaßt werden, wobei vorzugsweise eine Hybridisierungstemperatur von 58 °C und 34 Amplifizierungszyklen verwendet werden.

Der Mikrosatellit MS936FBN (=DIK4013) wird vorzugsweise bei der angegebenen Hybridisierungstemperatur in 36 Zyklen amplifiziert.

In Tabellen 1 und 2 werden Primer angegeben, mit denen diese bevorzugten Mikrosatelliten amplifizierbar sind. Dabei werden von den Seq. ID Nm 1- 56 jeweils eine gradzahlig bezeichnete Sequenz mit der nächst größeren ungradzahlig bezeichneten Sequenz paarweise kombiniert.

Die eingesetzte DNA wurde aus Blut- bzw. Haarproben mittels des QIAmp 96 Spin Blood Kits (Qiagen GmbH, Hilden) bzw. des Dneasy Tissue Kits (Qiagen GmbH, Hilden) isoliert. Die PCR wurde in 96 Mikrotiterplatten mit V-Boden und Deckel (BIOzym, Hessisch Oldendorf) im PCR-Cycler durchgeführt. Von der Rinder-DNA wurden 2 $\mu$L mit einer Konzentration von 10 ng/$\mu$L zu 10$\mu$L PCR-Mastermix gegeben. Die Zusammensetzung der PCR-Ansätze ist aus Tabelle 3 zu entnehmen.

Tabelle 3: bevorzugte Zusammensetzung der PCR-Ansätze

| Bestandteil | Mikrosatellit | | |
|---|---|---|---|
| | OARFCB5, BMS321, BP1, MS936FBN (Einzelansätze) | AGLA293[b], BL 23[b], BM2078[c], TGLA227[c], BMS2785[d], BM6507[d] ([b], [c], [d] in jeweils einem Ansatz) | RM103[a], BMS1315[a], BMC1009[a], BMS 1898[a] ([a] in einem Ansatz) |
| Wasser ($\mu$L, ultrarein) | 7,26 | 6,26 | 4,26 |
| 10x Puffer ($\mu$L, Q-Biogene) | 1,2 | 1,2 | 1,2 |
| DMSO, 99,5% ($\mu$L) | 0,24 | 0,24 | 0,24 |
| Vorwärtsprimer [10 pmol/$\mu$L] ($\mu$L) | 0,5 | Je 0,5 | Je 0,5 |
| Rückwärtsprimer [10 pmol/$\mu$L] ($\mu$L) | 0,5 | Je 0,5 | Je 0,5 |
| DNTPs [je 5 $\mu$M] ($\mu$L) | 0,2 | 0,2 | 0,2 |
| Taq-Polymerase [5 U/$\mu$L] ($\mu$L) | 0,1 | 0,1 | 0,1 |
| DNA [10 ng/$\mu$L] ($\mu$L) | 2 | 2 | 2 |

Die Bedingungen für die PCR waren 4 min bei 94 °C, dann Zyklen von 30 s bei 94 °C, 30 s bei der für die Primer spezifischen Hybridisierungstemperatur, 45 s bei 72 °C und nach Durchlaufen der Zyklen Kühlung bei 4 °C. Die Identifizierung der Ausprägungen, d.h. der Fragmentgrößen der PCR-Amplifikate, erfolgt durch Elektrophorese im 6% Polyacrylamid-Gel mit 5,1% Harnstoff.

Der Nachweis der Fragmente kann durch Identifikation von Farbstoffen mittels Laser erfolgen, die an zumindest einen der Primer gekoppelt sind Natürlich sind noch andere

Nachweisverfahren möglich, wie die unspezifische Markierung aller Amplifikate, beispielsweise durch Einsatz eines radioaktiv oder mehr auf andere Weise markierten Nukleotids, oder Primers, beispielsweise mit IRD-, Digoxigenin- oder Biotin-Markierung. Für die nachfolgenden Kopplungsanalysen wurden IRD-markierte Primer eingesetzt und zum Größenvergleich Längenstandards mit IRD-Markierung verwendet. Die optische Analyse erfolgte per Laserabtastung.

Die nachfolgenden Beispiele erläutern die Erfindung in größeren Einzelheiten an Hand einer Bullenfamilie. Die in Bezug genommenen Figuren zeigen die Analyse einzelner Mikrosatelliten:

- Figur 1 zeigt die optische Analyse im Polyacrylamid-Gel aufgetrennter Amplifikate, die mit Primern für den Mikrosatelliten TGLA227 erzeugt wurden,
- Figur 2 zeigt die Auswertung für die von BCSE betroffenen Tiere, die Nachkommen des Bullen Nr. 1 sind, hinsichtlich der Ausprägungen der Mikrosatelliten BMS2785, BM6507, TGLA227 und MS936FBN, die auf Chromosom Nr. 18 lokalisiert sind,

- Figur 3 zeigt die Auswertung gesunder Tiere, die ebenfalls Nachkommen des Bullen Nr. 1 sind, hinsichtlich der Ausprägungen der Mikrosatelliten BMS2785, BM6507, TGLA227 und MS936FBN, die auf Chromosom Nr. 18 lokalisiert sind,
- Figur 4 zeigt die Auswertung für die von BCSE betroffenen Tiere von Figur 2, die Nachkommen des Bullen Nr. 1 sind, hinsichtlich der Ausprägungen der Mikrosatelliten RM103, AGLA293, OarFCB5, BMS321, BMC1009 und BMS1898, die auf Chromosom Nr. 5 lokalisiert sind und
- Figur 5 zeigt die Auswertung für die gesunden Tiere von Figur 3, die ebenfalls Nachkommen des Bullen Nr. 1 sind, hinsichtlich der Ausprägungen der Mikrosatelliten RM103, AGLA293, OarFCB5, BMS321, BMC1009 und BMS1898, die auf Chromosom Nr. 18 lokalisiert sind.

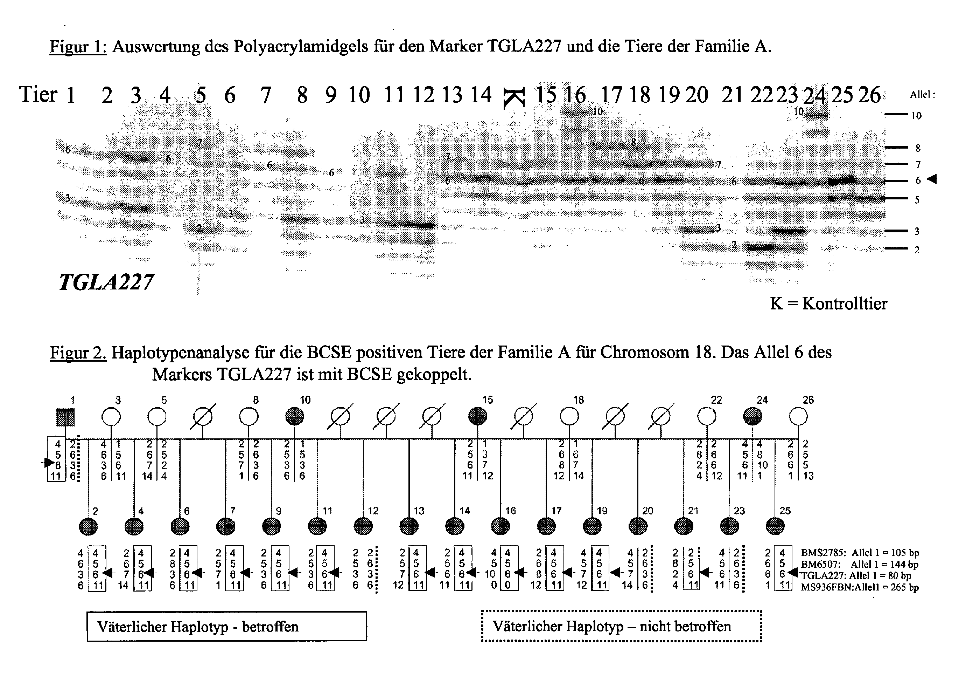

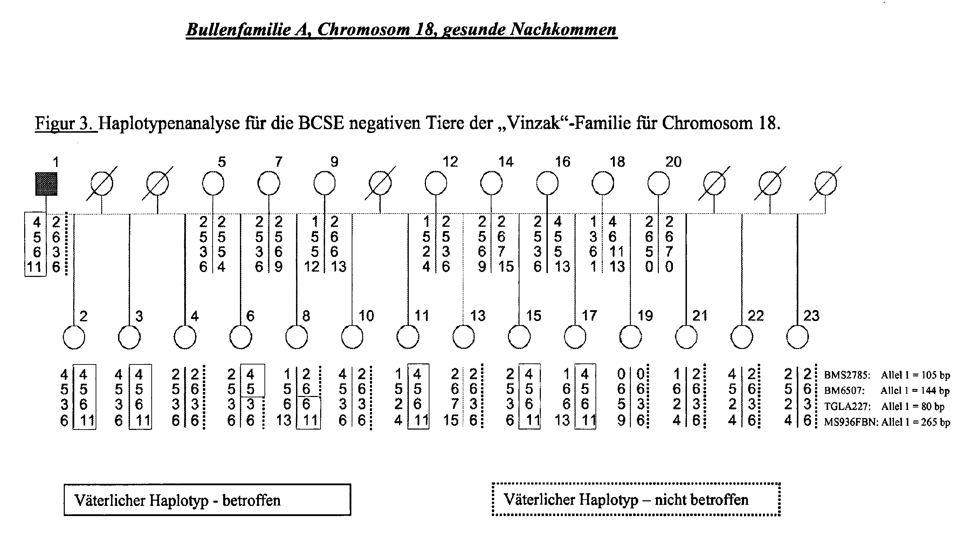

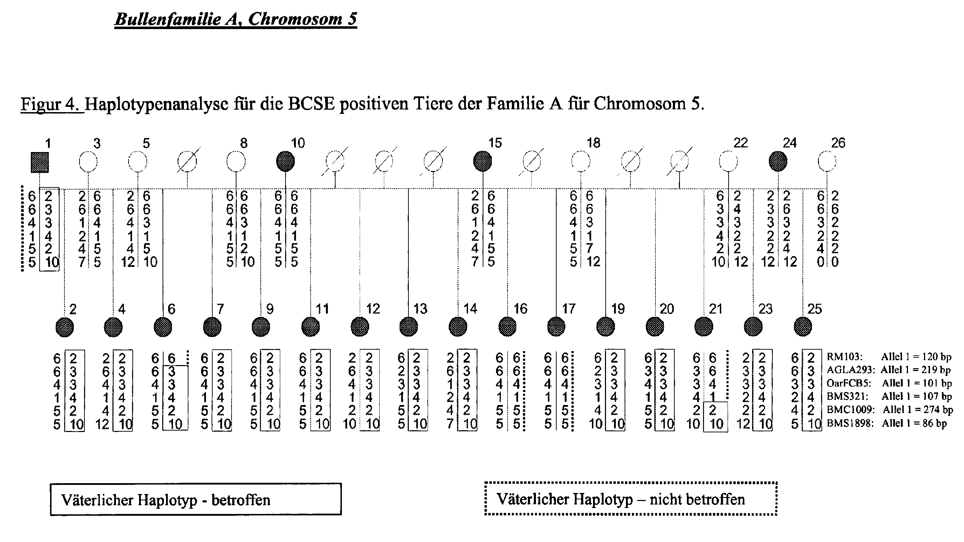

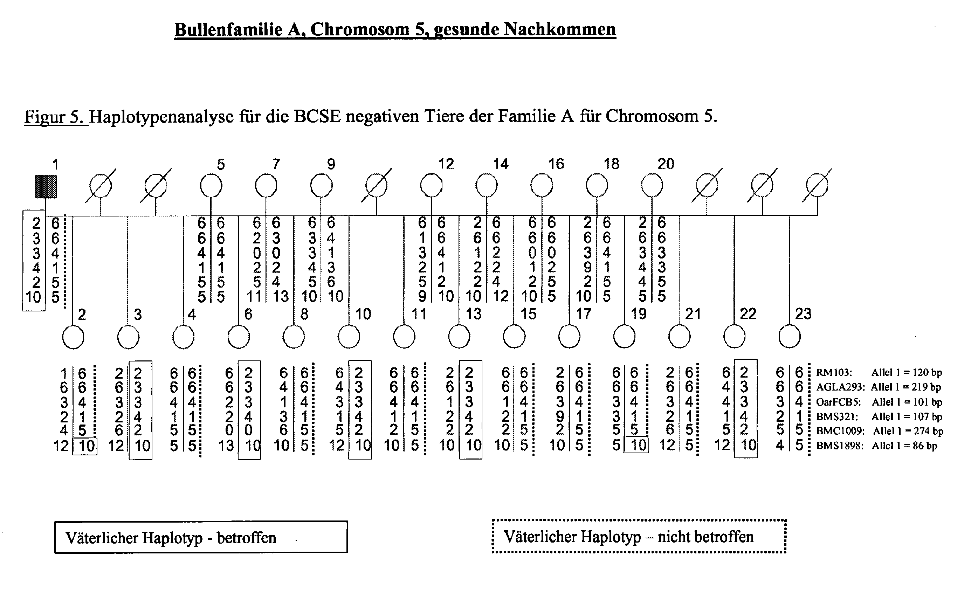

In Figuren 2 und 4 (BCSE betroffen) bzw. Figuren 3 und 5 (nicht BCSE betroffen) bezeichnen jeweils gleiche Ziffern identische Tiere, diese sind zwischen Figuren 2 und 3 bzw. 4 und 5 nicht identisch. Einzige Ausnahme ist der Bulle (Nr. 1), der jeweils (identisch) der Vater ist.

In den schematischen Darstellungen der Haplotypen geben die Ziffern links von der senkrechten Linie die Ausprägungen der jeweils analysierten Mikrosatelliten wieder, die vom Muttertier vererbt wurden, während die Ziffern rechts der senkrechten Linie diejenigen wiedergeben, die vom Vatertier stammen. Diese schematischen Stammbäume sind das Ergebnis einer Auswertung auf Basis einer Wahrscheinlichkeitsrechnung, wobei die Ziffern die Ausprägungen der jeweils analysierten Mikrosatelliten darstellen. Die Auswertung der Kopplung von beobachtetem Phänotyp und analysiertem Haplotyp zeigt, dass jeweils bestimmte Ausprägungen der Mikrosatelliten innerhalb eines Stammbaums ein Defektallel für BCSE anzeigen.

Bullen sind als ■ bzw. □ dargestellt, Kühe als • bzw. ○, wobei schwarze Symbole phänotypisch BCSE positive Tiere und offene Symbole phänotypisch negative Tiere bedeuten. Kühe mit unbekanntem Phänotyp sind als Ø dargestellt.

Die Ausprägungen der Mikrosatelliten, denen willkürlich aufsteigende Ziffern für zunehmende Länge des Amplifikats zugeordnet wurden, sind in der folgenden Tabelle 4 zusammengestellt:

Tabelle 4: Den Amplifikatlängen zugeordnete Ziffern in den Figuren

| Mikrosatellit | Ziffer des Allels in den Figuren | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| BMS2785 | 105 | 107 | 109 | 111 | 113 | 115 | 117 | 119 | 121 | 123 | 125 | 127 |
| BM6507 | 144 | 146 | 148 | 150 | 152 | 154 | 156 | 158 | 160 | 162 | 164 | 166 |
| TGLA227 | 80 | 82 | 84 | 86 | 88 | 90 | 92 | 94 | 96 | 98 | 100 | 102 |
| MS936FBN | 204 | 206 | 208 | 210 | 212 | 214 | 216 | 218 | 220 | 222 | 224 | 226 |
| RM103 | 120 | 122 | 124 | 126 | 128 | 130 | 132 | 134 | 136 | 138 | 140 | 142 |
| AGLA293 | 219 | 221 | 223 | 225 | 227 | 229 | 231 | 233 | 235 | 237 | 239 | 241 |
| OARFCB5 | 101 | 103 | 105 | 107 | 109 | 111 | 113 | 115 | 117 | 119 | 121 | 123 |
| BMS321 | 107 | 109 | 111 | 113 | 115 | 117 | 119 | 121 | 123 | 125 | 127 | 129 |
| BMC1009 | 274 | 276 | 278 | 280 | 282 | 284 | 286 | 288 | 290 | 292 | 294 | 296 |
| BMS1898 | 86 | 88 | 90 | 92 | 94 | 96 | 98 | 100 | 102 | 104 | 106 | 108 |

Die Kopplung kann mit statistischen Verfahren analysiert werden, vorliegend wurde das Verfahren von Abecasis et al. Nature Genetics 30, 97-101 (2002) in Form des Rechenprogramms MERLIN, Version 0.10.2 (Center for Stastical Genetics, University of Michigan, MI, USA) für die Berechnung von Zmean und deren Irrtumswahrscheinlichkeit verwendet. Der von Kong und Cox (American Journal of Human Genetics 61, 1179-1188 (1997)) aus Zmean abgeleitete Lod-Wert und dessen Irrtumswahrscheinlichkeit wurden gleichzeitig ermittelt. Diese statistische Analyse der Kopplungsbeziehungen zwischen dem phänotypischen Merkmal BCSE und genomweit verteilten, hochpolymorphen Mikrosatelliten ist ein nichtparametrisches Verfahren. Dieses benötigt im Gegensatz zu den parametrischen Verfahren der Kopplungsanalyse keine Annahmen hinsichtlich der Art des Erbgangs und der Allelfrequenz in der untersuchten Population. Das nichtparametrische Verfahren beruht auf der Schätzung des Anteils der gemeinsamen Allele, welche die betroffen Individuen innerhalb der jeweiligen Familien gemeinsam haben. Anschließend wird der Erwartungswert für den Anteil abstammungs-identischer Allele für jedes betroffene Familienmitglied berechnet und paarweise mit allen anderen betroffenen Familienmitgliedern verglichen. Die Abweichungen werden über alle paarweisen Vergleiche aufsummiert und in eine normalverteilte Variable mit Mittelwert Null und Standardabweichung 1 transformiert (Zmean). Dieser Wert stellt die Teststatistik dar, mit deren Hilfe eine signifikante Abweichung der Allelverteilung von dem erwarteten Anteil bei den gegebenen Verwandtschaftsbeziehungen zwischen den verglichenen verwandten Paaren mit BCSE und zufälliger Aufteilung der Allele festgestellt werden kann. Bei der Berechnung der Teststatistik werden alle Marker eines Chromosoms gleichzeitig miteinbezogen, wodurch die Anordnung der Allele auf dem jeweiligen Chromosomenstrang berücksichtigt wird und somit die Wahrscheinlichkeit steigt, eine signifikante Kopplung zu entdecken. Dementsprechend bezieht sich die Irrtumswahrscheinlichkeit auf alle Marker eines Chromosoms.

Für die Mikrosatelliten wurden die in Tabelle 5 dargestellten Werte für die Bullenfamilie A (Vinzak) bestimmt, wobei PIC als Berechnungswert für die Relevanz der Polymorphie (polymorphism information content) dient und errechnet wurde als

$$PIC = 1 - \sum_{i=1}^{n} p_i^2 - \sum_{i=1}^{n-1} \sum_{j=i+1}^{n} 2 p_i^2 p_j^2$$

mit $i \neq j$ oder i = j und
$p_i$ = Allelfrequenz für das i-te Allel des betreffenden Mikrosatellitenmarkers
$p_j$ = Allelfrequenz für das j-te Allel des betreffenden Mikrosatellitenmarkers
$\Sigma$ = Summation über die n bzw. n-1 Allele des betreffenden Mikrosatellitenmarkers.

Tabelle 5: Allelhäufigkeit, Heterozygotie und statistische Relevanz der Mikrosatelliten

| Mikrosatellit | Anzahl der Allele | Grad der Heterozygotie (%) | PIC* | Ziffer des Allels | | | | | | | | | |
|---|---|---|---|---|---|---|---|---|---|---|---|---|---|
| | | | | 1 | 2 | 3 | 4 | 5 | 6 | 7 | 8 | 9 | 10 |
| BP1 | 10 | 76 | 0,66 | 0,44 | 0,29 | 0,11 | 0,05 | 0,03 | 0,03 | 0,02 | 0,02 | 0,01 | 0,01 |
| BL23 | 3 | 28 | 0,23 | 0,86 | 0,11 | 0,03 | | | | | | | |
| RM103 | 3 | 49 | 0,40 | 0,59 | 0,38 | 0,03 | | | | | | | |
| AGLA293 | 5 | 39 | 0,44 | 0,71 | 0,16 | 0,07 | 0,05 | 0,01 | | | | | |
| BMS1315 | 4 | 32 | 0,28 | 0,83 | 0,09 | 0,08 | 0,01 | | | | | | |
| OarFCB5 | 4 | 66 | 0,60 | 0,47 | 0,30 | 0,17 | 0,06 | | | | | | |
| BMS321 | 5 | 67 | 0,57 | 0,49 | 0,33 | 0,11 | 0,05 | 0,02 | | | | | |
| BMC1009 | 6 | 69 | 0,62 | 0,43 | 0,27 | 0,24 | 0,03 | 0,02 | 0,01 | | | | |
| BMS1898 | 6 | 76 | 0,65 | 0,42 | 0,31 | 0,13 | 0,11 | 0,02 | 0,01 | | | | |
| BMS2785 | 3 | 44 | 0,42 | 0,69 | 0,21 | 0,10 | | | | | | | |
| MS936FBN | 9 | 81 | 0,80 | 0,25 | 0,25 | 0,14 | 0,13 | 0,10 | 0,06 | 0,04 | 0,02 | 0,01 | |
| RM2078 | 7 | 75 | 0,73 | 0,30 | 0,24 | 0,24 | 0,14 | 0,06 | 0,01 | 0,01 | | | |
| BM6507 | 4 | 60 | 0,50 | 0,55 | 0,33 | 0,10 | 0,01 | | | | | | |
| TGLA227 | 9 | 81 | 0,79 | 0,27 | 0,25 | 0,19 | 0,07 | 0,06 | 0,05 | 0,05 | 0,05 | 0,01 | |
| DIK2828 | 9 | 35 | 0,47 | 0,03 | 0,01 | 0,66 | 0,03 | 0,22 | 0,01 | 0,02 | 0,01 | 0,01 | |
| DIK4298 | 6 | 66 | 0,64 | 0,10 | 0,47 | 0,01 | 0,18 | 0,22 | 0,02 | | | | |
| DIK2718 | 6 | 74 | 0,67 | 0,08 | 0,05 | 0,03 | 0,24 | 0,15 | 0,45 | | | | |
| DIK2135 | 4 | 61 | 0,53 | 0,13 | 0,14 | 0,62 | 0,11 | | | | | | |
| DIK5002 | 6 | 66 | 0,59 | 0,12 | 0,04 | 0,11 | 0,15 | 0,57 | 0,01 | | | | |
| DIK2583 | 4 | 53 | 0,51 | 0,04 | 0,09 | 0,29 | 0,58 | | | | | | |
| DIK4759 | 7 | 66 | 0,57 | 0,12 | 0,01 | 0,06 | 0,59 | 0,15 | 0,05 | 0,02 | | | |
| DIK2465 | 6 | 50 | 0,44 | 0,03 | 0,33 | 0,61 | 0,01 | 0,01 | 0,01 | | | | |
| CSSM34 | 5 | 43 | 0,42 | 0,29 | 0,66 | 0,02 | 0,01 | 0,02 | | | | | |
| DIK4352 | 5 | 57 | 0,49 | 0,01 | 0,63 | 0,16 | 0,01 | 0,19 | | | | | |

(fortgesetzt)

| Mikrosatellit | Anzahl der Allele | Grad der Heterozygotie (%) | PIC* | Ziffer des Allels | | | | | | | | | |
|---|---|---|---|---|---|---|---|---|---|---|---|---|---|
| | | | | 1 | 2 | 3 | 4 | 5 | 6 | 7 | 8 | 9 | 10 |
| DIK2696 | 5 | 60 | 0,55 | 0,08 | 0,59 | 0,23 | 0,03 | 0,07 | | | | | |
| DIK5109 | 5 | 24 | 0,31 | 0,01 | 0,78 | 0,01 | 0,19 | 0,01 | | | | | |
| DIK4943 | 4 | 44 | 0,40 | 0,02 | 0,01 | 0,37 | 0,60 | | | | | | |
| DIK5235 | 4 | 46 | 0,42 | 0,32 | 0,62 | 0,02 | 0,04 | | | | | | |
| DIK4013 | 9 | 79 | 0,75 | 0,09 | 0,01 | 0,37 | 0,19 | 0,04 | 0,14 | 0,12 | 0,01 | 0,03 | |

## EP 1 659 184 B1

Für die Kopplungsanalyse ist es erforderlich, den Haplotyp der erfindungsgemäßen Mikrosatelliten zumindest einer Generation einer Rinderfamilie, beispielsweise mit einem gemeinsamen Elter, zu identifizieren. Denn aus einer Generation lassen sich die Haplotypen der Elterntiere rekonstruieren. Es ist jedoch bevorzugt, den Haplotyp der erfindungsgemäßen Mikrosatelliten zumindest eines Elters, bevorzugter beider Eltern, und Tieren der F1-Generation zu analysieren. Aus diesen Daten läßt sich die Kopplung erfindungsgemäß verwendeter Mikrosatelliten mit einem Defektallel für BCSE feststellen und für Nachkommen eine Voraussage treffen, ob diese ebenfalls Träger des Defektallels für BCSE sind. Die Ausprägung der Mikrosatelliten ist dabei jeweils familienspezifisch.

Beispiel 1: Analyse der Mikrosatelliten von Chromosomen Nr. 18 und Nr. 5 bei von BCSE betroffenen Tieren

In Figur 1 ist die Auftrennung von PCR-Amplifikaten des Mikrosatelliten TGLA227 im Harnstoff-Polyacrylamid-Gel dargestellt. Die Pfeile am rechten Rand bezeichnen ebenso wie die Ziffern innerhalb der Abbildung die Nummer des Allels, bzw. der Ausprägung dieses Mikrosatelliten. Die Ziffern sind willkürlich nach der Größe zugeordnet.

Figur 2 zeigt schematisch die Haplotypen einiger von BCSE betroffener Tiere der Bullenfamilie A (Bulle Vinzak). Die statistische Analyse für Chromosom Nr. 18 ergibt, dass bei den hier analysierten, für BCSE positiven Tieren der Bullenfamilie A das Allel Nr. 6 des Mikrosatelliten TGLA227 mit BCSE gekoppelt ist. Dieses Allel wird zusammen mit Allel 4 von BMS2785, Allel 5 von BM6507 sowie Allel 11 von MS936FBN vererbt. Auch diese Gruppierung von Allelen der Mikrosatelliten bleibt während des Erbgangs erhalten und zeigt die Kopplung mit dem Defektallel für BCSE.

Einzige Ausnahme ist die Kuh 21, bei der das Allel 2 von BMS2785 anstelle des bei den übrigen Tieren auftretenden väterlichen Allels 4 nachgewiesen wurde. Die Kuh 21 war die einzige Ausnahme der ansonsten engen Kopplung der erfindungsgemäßen Mikrosatelliten mit dem Genort, der für BCSE kodiert.

Figur 4 zeigt die Kopplungsanalyse für Mikrosatelliten von Chromosom Nr. 5 derselben Tiere wie Figur 2. Hier wurden die Mikrosatelliten RM103, AGLA293, OarFCB5, BMS321, BMC1009 und BMS1898 untersucht. Die statistische Auswertung mit dem Programm MERLIN zeigt bei Kuh 21, dass zumindest die Kombination des väterlichen Allels 2 von BMC1009 und Allel 10 von BMS1898 mit dem Defektallel für BCSE gekoppelt ist, Kuh 6 zeigt, dass auch die Kombination der väterlichen Allele 2 von BMC1009, Allel 10 von BMS1898, Allel 4 von BMS321, Allel 3 von OarFCB5 und Allel 3 von AGLA293 mit einem Defektallel für BCSE gekoppelt sind. Hingegen sind für diesen Stammbaum die väterlichen Allele 6 von RM103, Allel 6 von AGLA293, Allel 4 von OarFCB5 und Allel 1 von BMS321 nicht mit BCSE gekoppelt.

Die phänotypisch von BCSE betroffenen Tiere weisen für beide Chromosomen Mikrosatelliten auf, die mit einem Defektallel für BCSE gekoppelt sind. Bei den Tieren 16 und 17 liegen väterlicherseits Defektallele nur für Chromosom Nr. 18 vor, väterlicherseits trägt Chromosom Nr. 5 dieser Tiere kein Defektallel. Es ist daher zu vermuten, dass die Muttertiere von Tier 16 bzw. 17 diesen ein Defektallel auf Chromosom Nr. 5 vererbt haben.

Beispiel 2: Analyse der Mikrosatelliten von Chromosomen Nr. 18 und Nr. 5 bei gesunden Tieren

In Figur 3 ist schematisch die Analyse der Haplotypen von Tieren der Bullenfamilie A für andere Nachkommen gezeigt, die phänotypisch nicht von BCSE betroffen sind. Die Tiere 4, 6, 10, 13, 19, 21, 22 und 23 haben das Defektallel, das mit der Kombination der analysierten Mikrosatelliten gekoppelt ist, nämlich Allel 4 von BMS2785, Allel 5 von BM6507, Allel 6 von TGLA227 und Allel 11 von MS936FBN, vom Bullen nicht geerbt.

Die Tiere 2, 3, 8, 11, 15 und 17 könnten eventuell noch BCSE ausprägen, da sie den Haplotyp des Bullen aufweisen, der auf Chromosom Nr. 18 (siehe Figur 5) mit BCSE gekoppelt ist. Bis auf das Tier 3 scheint dies jedoch nicht wahrscheinlich, da nur dieses Tier sowohl an Chromosom Nr. 5 als auch Nr. 18 den für BCSE empfänglichen Haplotyp vom Bullen geerbt hat. Bei den Tieren 2, 8, 11, 15 und 17 liegt hingegen nur der mit BCSE gekoppelte Haplotyp auf Chromosom Nr. 18 vor und damit fehlt der zweite, für BCSE empfängliche Haplotyp von Chromosom Nr. 5.

**Patentansprüche**

1. Verwendung von Mikrosatelliten zur Bestimmung des Haplotyps eines Rindes in Bezug auf ein Defektallel, das für den phänotypischen Strabismus mit Exophthalmus (BCSE) ursächlich ist, wobei die Mikrosatelliten aus der Gruppe ausgewählt sind, die
BP1, **gekennzeichnet durch** die Amplifizierbarkeit aus genomischer DNA des Rindes durch das Primerpaar Seq.-ID Nr. 1 und Seq.-ID Nr. 2;
DIK2828, **gekennzeichnet durch** die Amplifizierbarkeit aus genomischer DNA des Rindes durch das Primerpaar Seq.-ID Nr. 3 und Seq.-ID Nr. 4 und Struktureinheit Seq.-ID Nr. 57; BL23, **gekennzeichnet durch** die Amplifizierbarkeit aus genomischer DNA des Rindes durch das Primerpaar Seq.-ID Nr. 5 und Seq.-ID Nr. 6;
RM1O3, **gekennzeichnet durch** die Struktureinheit Seq.-ID Nr. 58 und die Amplifizierbarkeit aus genomischer DNA des Rindes **durch** das Primerpaar Seq.-ID Nr. 7 und Seq.-ID Nr. 8;
DIK4298, **gekennzeichnet durch** die Struktureinheit Seq.-ID Nr. 59 und die Amplifizierbarkeit aus genomischer DNA des Rindes **durch** das Primerpaar Seq.-ID Nr. 9 und Seq.-ID Nr. 10;
DIK2718, **gekennzeichnet durch** die Struktureinheit Seq.-ID Nr. 60 und die Amplifizierbarkeit aus genomischer DNA des Rindes **durch** das Primerpaar Seq.-ID Nr. 11 und Seq.-ID Nr. 12;
DIK2135, **gekennzeichnet durch** die Struktureinheit Seq.-ID Nr. 61 und die Amplifizierbarkeit aus genomischer DNA des Rindes **durch** das Primerpaar Seq.-ID Nr. 13 und Seq.-ID Nr. 14;

AGLA293, **gekennzeichnet durch** die Amplifizierbarkeit aus genomischer DNA des Rindes **durch** das Primerpaar Seq.-ID Nr. 15 und Seq.-ID Nr. 16;

BMS 1315, **gekennzeichnet durch** die Struktureinheit Seq.-ID Nr. 62 und die Amplifizierbarkeit aus genomischer DNA des Rindes **durch** das Primerpaar Seq.-ID Nr. 17 und Seq.-ID Nr. 18;

DIK5002, **gekennzeichnet durch** die Struktureinheit Seq.-ID Nr. 63 und die Amplifizierbarkeit aus genomischer DNA des Rindes **durch** das Primerpaar Seq.-ID Nr. 19 und Seq.-ID Nr. 20;

OARFCB5, **gekennzeichnet durch** die Struktureinheit Seq.-ID Nr. 64 und die Amplifizierbarkeit aus genomischer DNA des Rindes **durch** das Primerpaar Seq.-ID Nr. 21 und Seq.-ID Nr. 22;

DIK2583, **gekennzeichnet durch** die Struktureinheit Seq.-ID Nr. 65 und die Amplifizierbarkeit aus genomische DNA des Rindes **durch** das Primerpaar Seq.-ID Nr. 23 und Seq.-ID Nr. 24;

BM321, **gekennzeichnet durch** die Struktureinheit Seq.-ID Nr. 66 und die Amplifizierbarkeit aus genomischer DNA des Rindes **durch** das Primerpaar Seq.-ID Nr. 25 und Seq.-ID Nr. 26;

DIK4759, **gekennzeichnet durch** die Struktureinheit Seq.-ID Nr. 67 und die Amplifizierbarkeit aus genomischer DNA des Rindes **durch** das Primerpaar Seq.-ID Nr. 27 und Seq.-ID Nr. 28;

BMC1009, **gekennzeichnet durch** die Amplifizierbarkeit aus genomischer DNA des Rindes durch das Primerpaar Seq.-ID Nr. 29 und Seq.-ID Nr. 30;

DIK2465, **gekennzeichnet durch** die Struktureinheit Seq.-ID Nr. 68 und die Amplifizierbarkeit aus genomischer DNA des Rindes **durch** das Primerpaar Seq.-ID Nr. 31 und Seq.-ID Nr. 32;

BMS 1898, **gekennzeichnet durch** die Struktureinheit Seq.-ID Nr. 69 und die Amplifizierbarkeit aus genomischer DNA des Rindes **durch** das Primerpaar Seq.-ID Nr. 33 und Seq.-ID Nr. 34;

CSSM34, **gekennzeichnet durch** die Struktureinheit Seq.-ID Nr. 70 und die Amplifizierbarkeit aus genomischer DNA des Rindes **durch** das Primerpaar Seq.-ID Nr. 35 und Seq.-ID Nr. 36;

DIK4352, **gekennzeichnet durch** die Struktureinheit Seq.-ID Nr. 71 und die Amplifizierbarkeit aus genomischer DNA des Rindes **durch** das Primerpaar Seq.-ID Nr. 37 und Seq.-ID Nr. 38;

BMS2785, **gekennzeichnet durch** die Struktureinheit Seq.-ID Nr. 72 und die Amplifizierbarkeit aus genomischer DNA des Rindes **durch** das Primerpaar Seq.-ID Nr. 39 und Seq.-ID Nr. 40;

DIK2696, **gekennzeichnet durch** die Struktureinheit Seq.-ID Nr. 73 und die Amplifizierbarkeit aus genomischer DNA des Rindes **durch** das Primerpaar Seq.-ID Nr. 41 und Seq.-ID Nr. 42;

BM2078, **gekennzeichnet durch** die Struktureinheit Seq.-ID Nr. 74 und die Amplifizierbarkeit aus genomischer DNA des Rindes **durch** das Primerpaar Seq.-ID Nr. 43 und Seq.-ID Nr. 44;

DIK5109, **gekennzeichnet durch** die Struktureinheit Seq.-ID Nr. 75 und die Amplifizierbarkeit aus genomischer DNA des Rindes **durch** das Primerpaar Seq.-ID Nr. 45 und Seq.-ID Nr. 46;

DIK4943, **gekennzeichnet durch** die Struktureinheit Seq.-ID Nr. 76 und die Amplifizierbarkeit aus genomischer DNA des Rindes **durch** das Primerpaar Seq.-ID Nr. 47 und Seq.-ID Nr. 48;

BM6507, **gekennzeichnet durch** die Struktureinheit Seq.-ID Nr. 77 und die Amplifizierbarkeit aus genomischer DNA des Rindes **durch** das Primerpaar Seq.-ID Nr. 49 und Seq.-ID Nr. 50;

DIK5235, **gekennzeichnet durch** die Struktureinheit Seq.-ID Nr. 78 und die Amplifizierbarkeit aus genomischer DNA des Rindes **durch** das Primerpaar Seq.-ID Nr. 51 und Seq.-ID Nr. 52;

TGLA227, **gekennzeichnet durch** die Amplifizierbarkeit aus genomischer DNA des Rindes durch das Primerpaar Seq.-ID Nr. 53 und Seq.-ID Nr. 54; und

MS936FBN, **gekennzeichnet durch** die Struktureinheit Seq.-ID Nr. 79 und die Amplifizierbarkeit aus genomischer DNA des Rindes **durch** das Primerpaar Seq.-ID Nr. 55 und Seq.-ID Nr. 56; umfasst

**2.** Verfahren zur Bestimmung des Haplotyps eines Rinds in Bezug auf ein Defektallel, das für den phänotypischen Strabismus mit Exophthalmus (BCSE) ursächlich ist, durch Verwendung nach einem der vorangehenden Ansprüche.

**3.** Verfahren nach Anspruch 2, bei dem mindestens zwei Mikrosatelliten verwendet werden.

**4.** Verwendung einzelsträngiger Nukleinsäuren nach Anspruch 1 oder in einem Verfahren nach einem der Ansprüche 2 oder 3, wobei die einzelsträngigen Nukleinsäuren aus der Gruppe ausgewählt sind, die die Seq.-ID Nrn. 1 bis 56 umfasst.

**Claims**

**1.** Use of microsatellites for the determination of the haplotype of a bovine in respect of a defect allele, which is causative of the phenotypic strabismus with exophtalmos (BCSE), wherein the microsatellites are selected from the group comprising

BP1, **characterized by** the capacity for amplification from genomic DNA of the bovine by the primer pair of Seq ID No. 1 and Seq ID No. 2;

DIK2828, **characterized by** the capacity for amplification from genomic DNA of the bovine by the primer pair of Seq ID No. 3 and Seq ID No. 4, and repeating unit Seq ID No. 57;

BL23, **characterized by** the capacity for amplification from genomic DNA of the bovine by the primer pair of Seq ID No.5 and Seq ID No. 6;

RM1O3, **characterized by** the repeating unit Seq ID No. 58 and the capacity for amplification from genomic DNA of the bovine by the primer pair of Seq ID No. 7 and Seq ID No. 8;

DIK4298, **characterized by** the repeating unit Seq ID No. 59 and the capacity for amplification from genomic DNA of the bovine by the primer pair of Seq ID No. 9 and Seq ID No. 10;

DIK2718, **characterized by** the repeating unit Seq ID No. 60 and the capacity for amplification from genomic DNA of the bovine by the primer pair of Seq ID No. 11 and Seq ID.No. 12;

DIK2135, **characterized by** the repeating unit Seq ID No. 61 and the capacity for amplification from genomic DNA of the bovine by the primer pair of Seq ID No. 13 and Seq ID No. 14;

AGLA293, **characterized by** the capacity for amplification from genomic DNA of the bovine by the primer pair of Seq ID No. 15 and Seq ID No. 16;

BMS 1315, **characterized by** the repeating unit Seq ID No. 62 and the capacity for amplification from genomic DNA of the bovine by the primer pair of Seq ID No. 17 and Seq ID No. 18;

DIK5002, **characterized by** the repeating unit Seq ID No. 63 and the capacity for amplification from genomic DNA of the bovine by the primer pair of Seq ID No. 19 and Seq ID No. 20;

OARFCB5, **characterized by** the repeating unit Seq ID No. 64 and the capacity for amplification from genomic DNA of the bovine by the primer pair of Seq ID No. 21 and Seq ID No. 22;

DIK2583, **characterized by** the repeating unit Seq ID No. 65 and the capacity for amplification from genomic DNA of the bovine by the primer pair of Seq ID No. 23 and Seq ID No. 24;

BM321, **characterized by** the repeating unit Seq ID No. 66 and the capacity for amplification from genomic DNA of the bovine by the primer pair of Seq ID No. 25 and Seq ID No. 26;

DIK4759, **characterized by** the repeating unit Seq ID No. 67 and the capacity for amplification from genomic DNA of the bovine by the primer pair of Seq ID No. 27 and Seq ID No. 28;

BMC1009, **characterized by** the capacity for amplification from genomic DNA of the bovine by the primer pair of Seq ID No. 29 and Seq ID No. 30;

DIK2465, **characterized by** the repeating unit Seq ID No. 68 and the capacity for amplification from genomic DNA of the bovine by the primer pair of Seq ID No. 31 and Seq ID No. 32;

BMS 1898, **characterized by** the repeating unit Seq ID No. 69 and the capacity for amplification from genomic DNA of the bovine by the primer pair of Seq ID No. 33 and Seq ID No. 34;

CSSM34, **characterized by** the repeating unit Seq ID No. 70 and the capacity for amplification from genomic DNA of the bovine by the primer pair of Seq ID No. 35 and Seq ID No. 36;

DIK4352, **characterized by** the repeating unit Seq ID No. 71 and the capacity for amplification from genomic DNA of the bovine by the primer pair of Seq ID No. 37 and Seq ID No. 38;

BMS2785, **characterized by** the repeating unit Seq ID No. 72 and the capacity for amplification from genomic DNA of the bovine by the primer pair of Seq ID No. 39 and Seq ID No. 40;

DIK2696, **characterized by** the repeating unit Seq ID No. 73 and the capacity for amplification from genomic DNA of the bovine by the primer pair of Seq ID No. 41 and Seq ID No. 42;

BM2078, **characterized by** the repeating unit Seq ID No. 74 and the capacity for amplification from genomic DNA of the bovine by the primer pair of Seq ID No. 43 and Seq ID No. 44;

DIK5109, **characterized by** the repeating unit Seq ID No. 75 and the capacity for amplification from genomic DNA of the bovine by the primer pair of Seq ID No. 45 and Seq ID No. 46;

DIK4943, **characterized by** the repeating unit Seq ID No. 76 and the capacity for amplification from genomic DNA of the bovine by the primer pair of Seq ID No. 47 and Seq ID No. 48;

BM6507, **characterized by** the repeating unit Seq ID No. 77 and the capacity for amplification from genomic DNA of the bovine by the primer pair of Seq ID No. 49 and Seq ID No. 50;

DIK5235, **characterized by** the repeating unit Seq ID No. 78 and the capacity for amplification from genomic DNA of the bovine by the primer pair of Seq ID No. 51 and Seq ID No. 52;

TGLA227, **characterized by** the capacity for amplification from genomic DNA of the bovine by the primer pair of Seq ID No. 53 and Seq ID No. 54;

MS936FBN, **characterized by** the repeating unit Seq ID No. 79 and the capacity for amplification from genomic DNA of the bovine by the primer pair of Seq ID No. 55 and Seq ID No. 56.

2. Process for the determination of the haplotype of a bovine in respect of a defect allele, which is causative of the phertotypic strabismus with exophtalmos (BCSE), by use according to one of the preceding claims.

3. Process according to claim 2, wherein at least two microsatellites are used.

4. Use of single-stranded nucleic acids according to claim 1 or in a process according to one of claims 2 or 3, wherein the single-stranded nucleic acids are selected from the group comprising Seq ID numbers 1 to 56.

**Revendications**

1. Utilisation de microsatellites afin de déterminer l'haplotype d'un boeuf par rapport à un allèle défectueux qui est la cause du strabisme phénotypique avec exophtalmie (BCSE), sachant que les microsatellites sont choisis dans un groupe, comprenant

   BP1, **caractérisé par** la capacité d'amplification à partir du DNA génomique du boeuf par la paire primaire ID de séquence N° 1 et ID de séquence N° 2 ;

   DIK2828, **caractérisé par** la capacité d'amplification à partir du DNA génomique du boeuf par la paire primaire ID de séquence N° 3 et ID de séquence N° 4 et l'unité de structure N° 57 ;

   BL23, **caractérisé par** la capacité d'amplification à partir du DNA génomique du boeuf par la paire primaire ID de séquence N° 5 et ID de séquence N° 6 ;

   RM1O3, **caractérisé par** l'unité de structure ID de séquence N° 58 et la capacité d'amplification à partir du DNA génomique du boeuf par la paire primaire ID de séquence N° 7 et ID de séquence N° 8 ;

   DIK4298, **caractérisé par** l'unité de structure ID de séquence N° 59 et la capacité d'amplification à partir du DNA génomique du boeuf par la paire primaire ID de séquence N° 9 et ID de séquence N° 10 ;

   BL2718, **caractérisé par** l'unité de structure ID de séquence N° 60 et la capacité d'amplification à partir du DNA génomique du boeuf par la paire primaire ID de séquence N° 11 et ID de séquence N° 12 ;

   BL2135, **caractérisé par** l'unité de structure ID de séquence N° 61 et la capacité d'amplification à partir du DNA génomique du boeuf par la paire primaire ID de séquence N° 13 et ID de séquence N° 14 ;

   AGLA293, **caractérisé par** la capacité d'amplification à partir du DNA génomique du boeuf par la paire primaire ID de séquence N° 15 et ID de séquence N° 16 ;

   BL1315, **caractérisé par** l'unité de structure ID de séquence N° 62 et la capacité d'amplification à partir du DNA génomique du boeuf par la paire primaire ID de séquence N° 17 et ID de séquence N° 18;

   DIK5002, **caractérisé par** l'unité de structure ID de séquence N° 63 et la capacité d'amplification à partir du DNA génomique du boeuf par la paire primaire ID de séquence N° 19 et ID de séquence N° 20 ;

   OARFCBS, **caractérisé par** l'unité de structure ID de séquence N° 64 et la capacité d'amplification à partir du DNA génomique du boeuf par la paire primaire ID de séquence N° 21 et ID de séquence N° 22 ;

   DIK2583, **caractérisé par** l'unité de structure ID de séquence N° 65 et la capacité d'amplification à partir du DNA génomique du boeuf par la paire primaire ID de séquence N° 23 et ID de séquence N° 24 ;

   BM321, **caractérisé par** l'unité de structure ID de séquence N° 66 et la capacité d'amplification à partir du DNA génomique du boeuf par la paire primaire ID de séquence N° 25 et ID de séquence N° 26 ;

   DIK4759, **caractérisé par** l'unité de structure ID de séquence N° 67 et la capacité d'amplification à partir du DNA génomique du boeuf par la paire primaire ID de séquence N° 27 et ID de séquence N° 28 ;

   BMC 1009, **caractérisé par** la capacité d'amplification à partir du DNA génomique du boeuf par la paire primaire ID de séquence N° 29 et ID de séquence N° 30 ;

   DIK2465, **caractérisé par** l'unité de structure ID de séquence N° 68 et la capacité d'amplification à partir du DNA génomique du boeuf par la paire primaire ID de séquence N° 31 et ID de séquence N° 32 ;

   BMS1898, **caractérisé par** l'unité de structure ID de séquence N° 69 et la capacité d'amplification à partir du DNA génomique du boeuf par la paire primaire ID de séquence N° 33 et ID de séquence N° 34 ;

   CSSM34, **caractérisé par** l'unité de structure ID de séquence N° 70 et la capacité d'amplification à partir du DNA génomique du boeuf par la paire primaire ID de séquence N° 35 et ID de séquence N° 36 ;

   DIK4352, **caractérisé par** l'unité de structure ID de séquence N° 71 et la capacité d'amplification à partir du DNA génomique du boeuf par la paire primaire ID de séquence N° 37 et ID de séquence N° 38 ;

   BMS2785, **caractérisé par** l'unité de structure ID de séquence N° 72 et la capacité d'amplification à partir du DNA génomique du boeuf par la paire primaire ID de séquence N° 39 et ID de séquence N° 40 ;

   DIK2696, **caractérisé par** l'unité de structure ID de séquence N° 73 et la capacité d'amplification à partir du DNA génomique du boeuf par la paire primaire ID de séquence N° 41 et ID de séquence N° 42 ;

   BM2078, **caractérisé par** l'unité de structure ID de séquence N° 74 et la capacité d'amplification à partir du DNA génomique du boeuf par la paire primaire ID de séquence N° 43 et ID de séquence N° 44 ;

   DIK5109, **caractérisé par** l'unité de structure ID de séquence N° 75 et la capacité d'amplification à partir du DNA génomique du boeuf par la paire primaire ID de séquence N° 45 et ID de séquence N° 46 ;

DIK4943, **caractérisé par** l'unité de structure ID de séquence N° 76 et la capacité d'amplification à partir du DNA génomique du boeuf par la paire primaire ID de séquence N° 47 et ID de séquence N° 48 ;

BM6507, **caractérisé par** l'unité de structure ID de séquence N° 77 et la capacité d'amplification à partir du DNA génomique du boeuf par la paire primaire ID de séquence N° 49 et ID de séquence N° 50 ;

DIK5235, **caractérisé par** l'unité de structure ID de séquence N° 78 et la capacité d'amplification à partir du DNA génomique du boeuf par la paire primaire ID de séquence N° 51 et ID de séquence N° 52 ;

TGLA227, **caractérisé par** la capacité d'amplification à partir du DNA génomique du boeuf par la paire primaire ID de séquence N° 53 et ID de séquence N° 54 ; et

MS936FBN, **caractérisé par** l'unité de structure ID de séquence N° 79 et la capacité d'amplification à partir du DNA génomique du boeuf par la paire primaire ID de séquence N° 55 et ID de séquence N° 56.

2. Procédé destiné à déterminer l'haplotype d'un boeuf par rapport à un allèle défectueux qui est la cause du strabisme phénotypique avec exophtalmie (BCSE) en employant une des revendications précédentes.

3. Procédé selon la revendication 2, pour lequel on emploie au moins deux microsatellites.

4. Utilisation d'acides nucléiques selon la revendication 3 ou un procédé selon les revendications 2 ou 3, sachant que les acides nucléiques sous forme de simple brin sont sélectionnés à partir d'un groupe qui comprend les ID de séquence N° 1 à 56.

Tier 1 2 3 4 5 6 7 8 9 10 11 12 13 14 K 15 16 17 18 19 20 21 22 23 24 25 26   Allel:

TGLA227

K = Kontrolltier

EP 1 659 184 B1

Figur 2. Haplotypenanalyse für die BCSE positiven Tiere der Familie A für Chromosom 18. Das Allel 6 des
Markers TGLA227 ist mit BCSE gekoppelt.

BMS2785: Allel 1 = 105 bp
BM6507:  Allel 1 = 144 bp
TGLA227: Allel 1 = 80 bp
MS936FBN: Allel 1 = 265 bp

Väterlicher Haplotyp - betroffen

Väterlicher Haplotyp – nicht betroffen

Figur 3. Haplotypenanalyse für die BCSE negativen Tiere der „Vinzak"-Familie für Chromosom 18.

BMS2785: Allel 1 = 105 bp
BM6507: Allel 1 = 144 bp
TGLA227: Allel 1 = 80 bp
MS936FBN: Allel 1 = 265 bp

Väterlicher Haplotyp - betroffen

Väterlicher Haplotyp – nicht betroffen

EP 1 659 184 B1

## *Bullenfamilie A, Chromosom 5*

Figur 4. Haplotypenanalyse für die BCSE positiven Tiere der Familie A für Chromosom 5.

| | |
|---|---|
| Väterlicher Haplotyp - betroffen | Väterlicher Haplotyp – nicht betroffen |

RM103: Allel 1 = 120 bp
AGLA293: Allel 1 = 219 bp
OarFCB5: Allel 1 = 101 bp
BMS321: Allel 1 = 107 bp
BMC1009: Allel 1 = 274 bp
BMS1898: Allel 1 = 86 bp

Figur 5. Haplotypenanalyse für die BCSE negativen Tiere der Familie A für Chromosom 5.

| Väterlicher Haplotyp - betroffen | Väterlicher Haplotyp – nicht betroffen |

RM103: Allel 1 = 120 bp
AGLA293: Allel 1 = 219 bp
OarFCB5: Allel 1 = 101 bp
BMS321: Allel 1 = 107 bp
BMC1009: Allel 1 = 274 bp
BMS1898: Allel 1 = 86 bp

EP 1 659 184 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0214544 A
- WO 9213102 A

**In der Beschreibung aufgeführte Nicht-Patentliteratur**

- **Bishop et al.** *Genetics,* 1994, vol. 136, 619-639
- **Stone et al.** *Mammalian Genome,* 1995, vol. 6, 714-724
- **Abecasis et al.** *Nature Genetics,* 2002, vol. 30, 97-101
- **Kong ; Cox.** *American Journal of Human Genetics,* 1997, vol. 61, 1179-1188

SEQUENCE LISTING

<110> Stiftung Tierärztliche Hochschule Hannover

<120> Genetischer Test auf Strabismus beim Rind

<130> N1004EP

<160> 79

<170> PatentIn version 3.1

<210> 1
<211> 22
<212> DNA
<213> artificial sequence

<220>
<223> forward primer / vorwärts Primer

<400> 1
aaaatccctt cataacagtg cc     22

<210> 2
<211> 20
<212> DNA
<213> artificial sequence

<220>
<223> backward primer / Rückwärts Primer

<400> 2
catcgtgaat tccagggttc     20

<210> 3
<211> 20
<212> DNA
<213> artificial sequence

<220>
<223> forward primer / vorwärts Primer

<400> 3
tggtgtctgc caaagcagta     20

<210> 4
<211> 20
<212> DNA
<213> artificial sequence

<220>
<223> backward primer / Rückwärts Primer

<400> 4
cgggaatttt cagagggaat     20

<210> 5
<211> 20
<212> DNA
<213> artificial sequence

<220>
<223> forward primer / vorwärts Primer

<400> 5
tcaatccctg ggtcaggaag     20

<210> 6
<211> 22
<212> DNA

<213> artificial sequence

<220>
<223> backward primer / Rückwärts Primer

<400> 6
cttatttcaa aattcggttg gg          22

<210> 7
<211> 24
<212> DNA
<213> artificial sequence

<220>
<223> forward primer / vorwärts Primer

<400> 7
tctgtgcact ttacatttaa caga          24

<210> 8
<211> 25
<212> DNA
<213> artificial sequence

<220>
<223> backward primer / Rückwärts Primer

<400> 8
gtggtctatt gaacttttgt tcaga          25

<210> 9
<211> 19
<212> DNA
<213> artificial sequence

<220>
<223> forward primer / vorwärts Primer

<400> 9
cccatgaccc aacaagcag          19

<210> 10
<211> 20
<212> DNA
<213> artificial sequence

<220>
<223> backward primer / Rückwärts Primer

<400> 10
gcctcagaga aatgggaaca          20

<210> 11
<211> 20
<212> DNA
<213> artificial sequence

<220>

<223> forward primer / vorwärts Primer

<400> 11
aggaaggaca aggacattgc        20

<210> 12
<211> 21
<212> DNA
<213> artificial sequence

<220>
<223> backward primer / Rückwärts Primer

<400> 12
agagggtcaa aggcttaatg g        21

<210> 13
<211> 20
<212> DNA
<213> artificial sequence

<220>
<223> forward primer / vorwärts Primer

<400> 13
acaatccatg gggtagcaaa        20

<210> 14
<211> 20
<212> DNA
<213> artificial sequence

<220>
<223> backward primer / Rückwärts Primer

<400> 14
tttggcactg caagaaagtg        20

<210> 15
<211> 25
<212> DNA
<213> artificial sequence

<220>
<223> forward primer / vorwärts Primer

<400> 15
gaaactcaac ccaagacaac tcaag        25

<210> 16
<211> 25
<212> DNA
<213> artificial sequence

<220>
<223> backward primer / Rückwärts Primer

<400> 16

atgactttat tctccaccta gcaga          25

<210> 17
<211> 22
<212> DNA
<213> artificial sequence

<220>
<223> forward primer / vorwärts Primer

<400> 17
aagccattga ttgtagattg gg          22

<210> 18
<211> 20
<212> DNA
<213> artificial sequence

<220>
<223> backward primer / Rückwärts Primer

<400> 18
gagtttcctt tttcccccac          20

<210> 19
<211> 20
<212> DNA
<213> artificial sequence

<220>
<223> forward primer / vorwärts Primer

<400> 19
tgtgctggag gtgatagctg          20

<210> 20
<211> 22
<212> DNA
<213> artificial sequence

<220>
<223> backward primer / Rückwärts Primer

<400> 20
tgcaggaata tgagagctga ga          22

<210> 21
<211> 25
<212> DNA
<213> artificial sequence

<220>
<223> forward primer / vorwärts Primer

<400> 21
gacctgaccc ttactctctt cactc          25

<210> 22

<211> 26
<212> DNA
<213> artificial sequence

<220>
<223> backward primer / Rückwärts Primer

<400> 22
aagttaattt tctggctgga aaaccc          26

<210> 23
<211> 20
<212> DNA
<213> artificial sequence

<220>
<223> forward primer / vorwärts Primer

<400> 23
ccagaggagg tggagagaca          20

<210> 24
<211> 20
<212> DNA
<213> artificial sequence

<220>
<223> backward primer / Rückwärts Primer

<400> 24
ccaaatatct cagggctgct          20

<210> 25
<211> 22
<212> DNA
<213> artificial sequence

<220>
<223> forward primer / vorwärts Primer

<400> 25
aagggtcaga caaaacttag ca          22

<210> 26
<211> 22
<212> DNA
<213> artificial sequence

<220>
<223> backward primer / Rückwärts Primer

<400> 26
atccttgccc taattctcat tc          22

<210> 27
<211> 20
<212> DNA
<213> artificial sequence

<220>
<223> forward primer / vorwärts Primer

<400> 27
agttggacct gccattgttc        20

<210> 28
<211> 19
<212> DNA
<213> artificial sequence

<220>
<223> backward primer / Rückwärts Primer

<400> 28
acttatgtgc gtgcgtgct       19

<210> 29
<211> 20
<212> DNA
<213> artificial sequence

<220>
<223> forward primer / vorwärts Primer

<400> 29
gcaccagcag agaggacatt        20

<210> 30
<211> 20
<212> DNA
<213> artificial sequence

<220>
<223> backward primer / Rückwärts Primer

<400> 30
accggctatt gtccatcttg        20

<210> 31
<211> 20
<212> DNA
<213> artificial sequence

<220>
<223> forward primer / vorwärts Primer

<400> 31
atgcaccaca gcgttcatag        20

<210> 32
<211> 22
<212> DNA
<213> artificial sequence

<220>
<223> backward primer / Rückwärts Primer

<400> 32
tcacgaagag aggctaacac tg          22


<210> 33
<211> 19
<212> DNA
<213> artificial sequence


<220>
<223> forward primer / vorwärts Primer


<400> 33
gacatcactt tggggcatg          19


<210> 34
<211> 19
<212> DNA
<213> artificial sequence


<220>
<223> backward primer / Rückwärts Primer


<400> 34
tctccccacc tcttccatc          19


<210> 35
<211> 20
<212> DNA
<213> artificial sequence


<220>
<223> forward primer / vorwärts Primer


<400> 35
caggccataa ctctgggact          20


<210> 36
<211> 20
<212> DNA
<213> artificial sequence


<220>
<223> backward primer / Rückwärts Primer


<400> 36
tcagccatct ctccttgtcc          20


<210> 37
<211> 20
<212> DNA
<213> artificial sequence


<220>
<223> forward primer / Vorwärts Primer


<400> 37
gttgttgcaa atggcatgat          20
<210> 38

<211> 20
<212> DNA
<213> artificial sequence

<220>
<223> backward primer / Rückwärts Primer

<400> 38
ggcatggaat tagggttgaa          20

<210> 39
<211> 18
<212> DNA
<213> artificial sequence

<220>
<223> forward primer / vorwärts Primer

<400> 39
acaaacctgt gcgccttg          18

<210> 40
<211> 19
<212> DNA
<213> artificial sequence

<220>
<223> backward primer / Rückwärts Primer

<400> 40
ggcaatcagt cggacacac          19

<210> 41
<211> 20
<212> DNA
<213> artificial sequence

<220>
<223> forward primer / vorwärts Primer

<400> 41
gtggggaaca ggttgttgaa          20

<210> 42
<211> 20
<212> DNA
<213> artificial sequence

<220>
<223> backward primer / Rückwärts Primer

<400> 42
acccactcca ctgttcttgc          20

<210> 43
<211> 19
<212> DNA
<213> artificial sequence

<220>
<223> forward primer / vorwärts Primer

<400> 43
cccaaaagaa gccaggaag          19

<210> 44
<211> 20
<212> DNA
<213> artificial sequence

<220>
<223> backward primer / Rückwärts Primer

<400> 44
tcagagtttg gggtcctcag          20

<210> 45
<211> 20
<212> DNA
<213> artificial sequence

<220>
<223> forward primer / vorwärts Primer

<400> 45
gcacagagat gctcggtctc          20

<210> 46
<211> 20
<212> DNA
<213> artificial sequence

<220>
<223> backward primer / Rückwärts Primer

<400> 46
gctcagatcc tccgcactta          20

<210> 47
<211> 20
<212> DNA
<213> artificial sequence

<220>
<223> forward primer / vorwärts Primer

<400> 47
agacagggcc tgatgaatgt          20

<210> 48
<211> 20
<212> DNA
<213> artificial sequence

<220>
<223> backward primer / Rückwärts Primer

<400> 48
cccacctctc acaacacaca          20


<210> 49
<211> 22
<212> DNA
<213> artificial sequence


<220>
<223> forward primer / vorwärts Primer


<400> 49
acttagcaca atgccctcta gg          22


<210> 50
<211> 22
<212> DNA
<213> artificial sequence


<220>
<223> backward primer / Rückwärts Primer


<400> 50
atgttattcc atcaggagga gc          22


<210> 51
<211> 20
<212> DNA
<213> artificial sequence


<220>
<223> forward primer / vorwärts Primer


<400> 51
tcgtgtgctg ttcattgtca          20


<210> 52
<211> 20
<212> DNA
<213> artificial sequence


<220>
<223> backward primer / Rückwärts Primer


<400> 52
tcagcaggca ggtcctttat          20


<210> 53
<211> 25
<212> DNA
<213> artificial sequence


<220>
<223> forward primer / vorwärts Primer


<400> 53
cgaattccaa atctgttaat ttgct          25

<210> 54
<211> 24
<212> DNA
<213> artificial sequence

<220>
<223> backward primer / Rückwärts Primer

<400> 54
acagacagaa actcaatgaa agca          24

<210> 55
<211> 20
<212> DNA
<213> artificial sequence

<220>
<223> forward primer / vorwärts Primer

<400> 55
gaaatttgtg acccctgcat          20

<210> 56
<211> 20
<212> DNA
<213> artificial sequence

<220>
<223> backward primer / Rückwärts Primer

<400> 56
ctaaagctct gcctcccaag          20

<210> 57
<211> 2
<212> DNA
<213> repeating unit of microsatellite / Struktureinheit des Mikrosatelliten

<220>
<221> repeat_unit
<222> (1)..(2)
<223>

<400> 57
ca          2

<210> 58
<211> 2
<212> DNA
<213> repeating unit of microsatellite / Struktureinheit des Mikrosatelliten

<220>
<221> repeat_unit
<222> (1)..(2)
<223>

<400> 58
ca 2

```
<210> 59
<211>      2
<212> DNA
<213> repeating unit of microsatellite / Struktureinheit des Mikrosatelliten

<220>
<221> repeat_unit
<222> (1)..(2)
<223>

<400> 59
tg        2

<210> 60
<211> 2
<212> DNA
<213> repeating unit of microsatellite / Struktureinheit des Mikrosatelliten

<220>
<221> repeat_unit
<222> (1)..(2)
<223>

<400> 60
tg        2

<210> 61
<211> 2
<212> DNA
<213> repeating unit of microsatellite / Struktureinheit des Mikrosatelliten

<220>
<221> repeat_unit
<222> (1)..(2)
<223>

<400> 61
tg        2

<210> 62
<211> 2
<212> DNA
<213> repeating unit of microsatellite / Struktureinheit des Mikrosatelliten

<220>
<221> repeat_unit
<222> (1)..(2)
<223>

<400> 62
tg 2

<210> 63
<211> 2
<212> DNA
<213> repeating unit of microsatellite / Struktureinheit des Mikrosatelliten

<220>
```

<221> repeat_unit
<222> (1)..(2)
<223>

<400> 63
tg        2

<210> 64
<211> 2
<212> DNA
<213> repeating unit of microsatellite / Struktureinheit des Mikrosatelliten

<220>
<221> repeat_unit
<222> (1)..(2)
<223>

<400> 64
tg 2

<210> 65
<211>         2
<212> DNA
<213> repeating unit of microsatellite / Struktureinheit des Mikrosatelliten

<220>
<221> repeat_unit
<222> (1) .. (2)
<223>

<400> 65
ca         2

<210> 66
<211> 2
<212> DNA
<213> repeating unit of microsatellite / Struktureinheit des Mikrosatelliten

<220>
<221> repeat_unit
<222> (1)..(2)
<223>

<400> 66
ca        2

<210> 67
<211> 2
<212> DNA
<213> repeating unit of microsatellite / Struktureinheit des Mikrosatelliten

<220>
<221> repeat_unit
<222> (1)..(2)
<223>

<400> 67
tg        2

<210> 68
<211> 2
<212> DNA
<213> repeating unit of microsatellite / Struktureinheit des Mikrosatelliten

<220>
<221> repeat_unit
<222> (1)..(2)
<223>

<400> 68
ca            2

<210> 69
<211> 2
<212> DNA
<213> repeating unit of microsatellite / Struktureinheit des Mikrosatelliten

<220>
<221> repeat_unit
<222> (1) .. (2)
<223>

<400> 69
tg            2

<210> 70
<211> 2
<212> DNA
<213> repeating unit of microsatellite / Struktureinheit des Mikrosatelliten

<220>
<221> repeat_unit
<222> (1) .. (2)
<223>

<400> 70
tg            2

<210> 71
<211> 2
<212> DNA
<213> repeating unit of microsatellite / Struktureinheit des Mikrosatelliten

<220>
<221> repeat_unit
<222> (1)..(2)
<223>

<400> 71
tg            2

<210> 72
<211> 2
<212> DNA
<213> repeating unit of microsatellite / Struktureinheit des Mikrosatelliten

<220>

<221> repeat_unit
<222> (1)..(2)
<223>

<400> 72
tg        2

<210> 73
<211> 2
<212> DNA
<213> repeating unit of microsatellite / Struktureinheit des Mikrosatelliten

<220>
<221> repeat_unit
<222> (1)..(2)
<223>

<400> 73
tg        2

<210> 74
<211> 2
<212> DNA
<213> repeating unit of microsatellite / Struktureinheit des Mikrosatelliten

<220>
<221> repeat_unit
<222> (1) .. (2)
<223>

<400> 74
ca        2

<210> 75
<211> 2
<212> DNA
<213> repeating unit of microsatellite / Struktureinheit des Mikrosatelliten

<220>
<221> repeat_unit
<222> (1) .. (2)
<223>

<400> 75
tg        2

<210> 76
<211> 2
<212> DNA
<213> repeating unit of microsatellite / Struktureinheit des Mikrosatelliten

<220>
<221> repeat_unit
<222> (1)..(2)
<223>

<400> 76
tg        2

<210> 77
<211> 2
<212> DNA
<213> repeating unit of microsatellite /Struktureinheit des Mikrosatelliten

<220>
<221> repeat_unit
<222> (1)..(2)
<223>

<400> 77
ca          2

<210> 78
<211> 2
<212> DNA
<213> repeating unit of microsatellite / Struktureinheit des Mikrosatelliten

<220>
<221> repeat_unit
<222> (1)..(2)
<223>

<400> 78
ca          2

<210> 79
<211> 2
<212> DNA
<213> repeating unit of microsatellite / Struktureinheit des Mikrosatelliten

<220>
<221> repeat_unit
<222> (1)..(2)
<223>

<400> 79
tg          2

SEQUENCE LISTING

<110> Stiftung Tierärztliche Hochschule Hannover

<120> Genetischer Test auf strabismus beim Rind

<130> N1004EP

<160> 79

<170> PatentIn version 3.1

<210> 1
<211> 22
<212> DNA
<213> artificial sequence

<220>
<223> forward primer / vorwärts Primer

<400> 1
aaaatccctt cataacagtg cc          22

<210> 2
<211> 20
<212> DNA
<213> artificial sequence

<220>
<223> backward primer / Rückwärts Primer

<400> 2
catcgtgaat tccagggttc          20

<210> 3
<211> 20
<212> DNA
<213> artificial sequence

<220>

<223> forward primer / vorwärts Primer

<400> 3
tggtgtctgc caaagcagta          20

<210> 4
<211> 20
<212> DNA
<213> artificial sequence

<220>
<223> backward primer / Rückwärts Primer

<400> 4
cgggaatttt cagagggaat          20

<210> 5
<211> 20
<212> DNA
<213> artificial sequence

<220>
<223> forward primer / vorwärts Primer

<400> 5
tcaatccctg ggtcaggaag          20

<210> 6
<211> 22
<212> DNA
<213> artificial sequence

<220>
<223> backward primer / Rückwärts Primer

<400> 6
cttatttcaa aattcggttg gg      22

<210> 7
<211> 24
<212> DNA
<213> artificial sequence

<220>
<223> forward primer / vorwärts Primer

<400> 7
tctgtgcact ttacatttaa caga      24

<210> 8
<211> 25
<212> DNA
<213> artificial sequence

<220>
<223> backward primer / Rückwärts Primer

<400> 8
gtggtctatt gaacttttgt tcaga      25

<210> 9
<211> 19
<212> DNA
<213> artificial sequence

<220>
<223> forward primer / vorwärts Primer

<400> 9
cccatgaccc aacaagcag      19

<210> 10
<211> 20
<212> DNA
<213> artificial sequence

<220>
<223> backward primer / Rückwärts Primer

<400> 10
gcctcagaga aatgggaaca      20

<210> 11
<211> 20
<212> DNA
<213> artificial sequence

<220>
<223> forward primer / vorwärts Primer

<400> 11
aggaaggaca aggacattgc      20

<210> 12
<211> 21
<212> DNA
<213> artificial sequence

<220>
<223> backward primer / Rückwärts Primer

<400> 12
agagggtcaa aggcttaatg g          21

<210> 13
<211> 20
<212> DNA
<213> artificial sequence

<220>
<223> forward primer / vorwärts Primer

<400> 13
acaatccatg gggtagcaaa          20

<210> 14
<211> 20
<212> DNA
<213> artificial sequence

<220>
<223> backward primer / Rückwärts Primer

<400> 14
tttggcactg caagaaagtg          20
<210> 15
<211> 25
<212> DNA
<213> artificial sequence

<220>
<223> forward primer / vorwärts Primer

<400> 15
gaaactcaac ccaagacaac tcaag          25

<210> 16
<211> 25
<212> DNA
<213> artificial sequence

<220>
<223> backward primer / Rückwärts Primer

<400> 16
atgactttat tctccaccta gcaga          25

<210> 17
<211> 22
<212> DNA
<213> artificial sequence

<220>
<223> forward primer / vorwärts Primer

<400> 17
aagccattga ttgtagattg gg        22


<210> 18
<211> 20
<212> DNA
<213> artificial sequence

<220>
<223> backward primer / Rückwärts Primer

<400> 18
gagtttcctt tttcccccac        20


<210> 19
<211> 20
<212> DNA
<213> artificial sequence

<220>
<223> forward primer / vorwärts Primer

<400> 19
tgtgctggag gtgatagctg        20


<210> 20
<211> 22
<212> DNA
<213> artificial sequence

<220>
<223> backward primer / Rückwärts Primer

<400> 20
tgcaggaata tgagagctga ga        22


<210> 21
<211> 25
<212> DNA
<213> artificial sequence

<220>
<223> forward primer / vorwärts Primer

<400> 21
gacctgaccc ttactctctt cactc        25


<210> 22
<211> 26
<212> DNA
<213> artificial sequence

<220>
<223> backward primer / Rückwärts Primer

<400> 22
aagttaattt tctggctgga aaaccc        26


<210> 23
<211> 20
<212> DNA
<213> artificial sequence


<220>
<223> forward primer / vorwärts Primer


<400> 23
ccagaggagg tggagagaca        20


<210> 24
<211> 20
<212> DNA
<213> artificial sequence


<220>
<223> backward primer / Rückwärts Primer


<400> 24
ccaaatatct cagggctgct        20


<210> 25
<211> 22
<212> DNA
<213> artificial sequence


<220>
<223> forward primer / vorwärts Primer


<400> 25
aagggtcaga caaaacttag ca        22


<210> 26
<211> 22
<212> DNA
<213> artificial sequence


<220>
<223> backward primer / Rückwärts Primer


<400> 26
atccttgccc taattctcat tc        22


<210> 27
<211> 20
<212> DNA
<213> artificial sequence


<220>
<223> forward primer / vorwärts Primer


<400> 27
agttggacct gccattgttc        20

<210> 28
<211> 19
<212> DNA
<213> artificial sequence

<220>
<223> backward primer / Rückwärts Primer

<400> 28
acttatgtgc gtgcgtgct          19

<210> 29
<211> 20
<212> DNA
<213> artificial sequence

<220>
<223> forward primer / vorwärts Primer

<400> 29
gcaccagcag agaggacatt          20

<210> 30
<211> 20
<212> DNA
<213> artificial sequence

<220>
<223> backward primer / Rückwärts Primer

<400> 30
accggctatt gtccatcttg          20

<210> 31
<211> 20
<212> DNA
<213> artificial sequence

<220>
<223> forward primer / vorwärts Primer

<400> 31
atgcaccaca gcgttcatag          20

<210> 32
<211> 22
<212> DNA
<213> artificial sequence

<220>
<223> backward primer / Rückwärts Primer

<400> 32
tcacgaagag aggctaacac tg          22

<210> 33
<211> 19
<212> DNA

<213> artificial sequence

<220>
<223> forward primer / vorwärts Primer

<400> 33
gacatcactt tggggcatg        19

<210> 34
<211> 19
<212> DNA
<213> artificial sequence

<220>
<223> backward primer / Rückwärts Primer

<400> 34
tctccccacc tcttccatc        19

<210> 35
<211> 20
<212> DNA
<213> artificial sequence

<220>
<223> forward primer / vorwärts Primer

<400> 35
caggccataa ctctgggact        20

<210> 36
<211> 20
<212> DNA
<213> artificial sequence

<220>
<223> backward primer / Rückwärts Primer

<400> 36
tcagccatct ctccttgtcc        20

<210> 37
<211> 20
<212> DNA
<213> artificial sequence

<220>
<223> forward primer / vorwärts Primer

<400> 37
gttgttgcaa atggcatgat        20

<210> 38
<211> 20
<212> DNA
<213> artificial sequence

<220>

<223> backward primer / Rückwärts Primer

<400> 38
ggcatggaat tagggttgaa          20

<210> 39
<211> 18
<212> DNA
<213> artificial sequence

<220>
<223> forward primer / vorwärts Primer

<400> 39
acaaacctgt gcgccttg          18

<210> 40
<211> 19
<212> DNA
<213> artificial sequence

<220>
<223> backward primer / Rückwärts Primer

<400> 40
ggcaatcagt cggacacac          19

<210> 41
<211> 20
<212> DNA
<213> artificial sequence

<220>
<223> forward primer / vorwärts Primer

<400> 41
gtggggaaca ggttgttgaa          20

<210> 42
<211> 20
<212> DNA
<213> artificial sequence

<220>
<223> backward primer / Rückwärts Primer

<400> 42
acccactcca ctgttcttgc          20

<210> 43
<211> 19
<212> DNA
<213> artificial sequence

<220>
<223> forward primer / vorwärts Primer

<400> 43

cccaaaagaa gccaggaag        19

<210> 44
<211> 20
<212> DNA
<213> artificial sequence

<220>
<223> backward primer / Rückwärts Primer

<400> 44
tcagagtttg gggtcctcag        20

<210> 45
<211> 20
<212> DNA
<213> artificial sequence

<220>
<223> forward primer / vorwärts Primer

<400> 45
gcacagagat gctcggtctc        20

<210> 46
<211> 20
<212> DNA
<213> artificial sequence

<220>
<223> backward primer / Rückwärts Primer

<400> 46
gctcagatcc tccgcactta        20

<210> 47
<211> 20
<212> DNA
<213> artificial sequence

<220>
<223> forward primer / vorwärts Primer

<400> 47
agacagggcc tgatgaatgt        20

<210> 48
<211> 20
<212> DNA
<213> artificial sequence

<220>
<223> backward primer / Rückwärts Primer

<400> 48
cccacctctc acaacacaca        20

<210> 49

<211> 22
<212> DNA
<213> artificial sequence

<220>
<223> forward primer / vorwärts Primer

<400> 49
acttagcaca atgccctcta gg       22

<210> 50
<211> 22
<212> DNA
<213> artificial sequence

<220>
<223> backward primer / Rückwärts Primer

<400> 50
atgttattcc atcaggagga gc       22

<210> 51
<211> 20
<212> DNA
<213> artificial sequence

<220>

<223> forward primer / vorwärts Primer

<400> 51
tcgtgtgctg ttcattgtca       20

<210> 52
<211> 20
<212> DNA
<213> artificial sequence

<220>
<223> backward primer / Rückwärts Primer

<400> 52
tcagcaggca ggtcctttat       20

<210> 53
<211> 25
<212> DNA
<213> artificial sequence

<220>
<223> forward primer / vorwärts Primer

<400> 53
cgaattccaa atctgttaat ttgct       25

<210> 54
<211> 24
<212> DNA

<213> artificial sequence

<220>
<223> backward primer / Rückwärts Primer

<400> 54
acagacagaa actcaatgaa agca          24

<210> 55
<211> 20
<212> DNA
<213> artificial sequence

<220>
<223> forward primer / vorwärts Primer

<400> 55
gaaatttgtg acccctgcat          20

<210> 56
<211> 20
<212> DNA
<213> artificial sequence

<220>
<223> backward primer / Rückwärts Primer

<400> 56
ctaaagctct gcctcccaag          20

<210> 57
<211> 2
<212> DNA
<213> repeating unit of microsatellite / Struktureinheit des Mikrosatelliten

<220>
<221> repeat_unit
<222> (1)..(2)
<223>

<400> 57
ca          2

<210> 58
<211> 2
<212> DNA
<213> repeating unit of microsatellite / Struktureinheit des Mikrosatelliten

<220>
<221> repeat_unit
<222> (1)..(2)
<223>

<400> 58
ca          2

<210> 59
<211> 2

<212> DNA
<213> repeating unit of microsatellite / Struktureinheit des Mikrosatelliten

<220>
<221> repeat_unit
<222> (1)..(2)
<223>

<400> 59
tg          2

<210> 60
<211> 2
<212> DNA
<213> repeating unit of microsatellite / Struktureinheit des Mikrosatelliten

<220>
<221> repeat_unit
<222> (1)..(2)
<223>

<400> 60
tg          2

<210> 61
<211> 2
<212> DNA
<213> repeating unit of microsatellite / Struktureinheit des Mikrosatelliten

<220>
<221> repeat_unit
<222> (1)..(2)
<223>

<400> 61
tg          2

<210> 62
<211> 2
<212> DNA
<213> repeating unit of microsatellite / Struktureinheit des Mikrosatelliten

<220>
<221> repeat_unit
<222> (1)..(2)
<223>

<400> 62
tg          2

<210> 63
<211> 2
<212> DNA
<213> repeating unit of microsatellite / Struktureinheit des Mikrosatelliten

<220>
<221> repeat_unit
<222> (1)..(2)

<223>

<400> 63
tg        2

<210> 64
<211> 2
<212> DNA
<213> repeating unit of microsatellite / Struktureinheit des Mikrosatelliten

<220>
<221> repeat_unit
<222> (1)..(2)
<223>

<400> 64
tg        2

<210> 65
<211> 2
<212> DNA
<213> repeating unit of microsatellite / Struktureinheit des Mikrosatelliten

<220>
<221> repeat_unit
<222> (1)..(2)
<223>

<400> 65
ca        2

<210> 66
<211> 2
<212> DNA
<213> repeating unit of microsatellite / Struktureinheit des Mikrosatelliten

<220>
<221> repeat_unit
<222> (1)..(2)
<223>

<400> 66
ca        2

<210> 67
<211> 2
<212> DNA
<213> repeating unit of microsatellite / Struktureinheit des Mikrosatelliten

<220>
<221> repeat_unit
<222> (1)..(2)
<223>

<400> 67
tg        2

<210> 68

<211> 2
<212> DNA
<213> repeating unit of microsatellite / Struktureinheit des Mikrosatelliten

<220>
<221> repeat_unit
<222> (1)..(2)
<223>

<400> 68
ca          2

<210> 69
<211> 2
<212> DNA
<213> repeating unit of microsatellite / Struktureinheit des Mikrosatelliten

<220>
<221> repeat_unit
<222> (1)..(2)
<223>

<400> 69
tg          2

<210> 70
<211> 2
<212> DNA
<213> repeating unit of microsatellite / Struktureinheit des Mikrosatelliten

<220>
<221> repeat_unit
<222> (1)..(2)
<223>

<400> 70
tg          2

<210> 71
<211> 2
<212> DNA
<213> repeating unit of microsatellite / Struktureinheit des Mikrosatelliten

<220>
<221> repeat_unit
<222> (1)..(2)
<223>

<400> 71
tg          2

<210> 72
<211> 2
<212> DNA
<213> repeating unit of microsatellite / Struktureinheit des Mikrosatelliten

<220>
<221> repeat_unit

<222> (1)..(2)
<223>

<400> 72
tg          2

<210> 73
<211> 2
<212> DNA
<213> repeating unit of microsatellite / Struktureinheit des Mikrosatelliten

<220>
<221> repeat_unit
<222> (1)..(2)
<223>

<400> 73
tg          2

<210> 74
<211> 2
<212> DNA
<213> repeating unit of microsatellite / Struktureinheit des Mikrosatelliten

<220>
<221> repeat_unit
<222> (1)..(2)
<223>

<400> 74
ca          2

<210> 75
<211> 2
<212> DNA
<213> repeating unit of microsatellite / Struktureinheit des Mikrosatelliten

<220>
<221> repeat_unit
<222> (1)..(2)
<223>

<400> 75
tg          2

<210> 76
<211> 2
<212> DNA
<213> repeating unit of microsatellite / Struktureinheit des Mikrosatelliten

<220>
<221> repeat_unit
<222> (1)..(2)
<223>

<400> 76
tg          2

EP 1 659 184 B1

<210> 77
<211> 2
<212> DNA
<213> repeating unit of microsatellite / Struktureinheit des Mikrosatelliten

<220>
<221> repeat_unit
<222> (1)..(2)
<223>

<400> 77
ca          2

<210> 78
<211> 2
<212> DNA
<213> repeating unit of microsatellite / Struktureinheit des Mikrosatelliten

<220>
<221> repeat_unit
<222> (1)..(2)
<223>

<400> 78
ca          2

<210> 79
<211> 2
<212> DNA
<213> repeating unit of microsatellite / Struktureinheit des Mikrosatelliten

<220>
<221> repeat_unit
<222> (1)..(2)
<223>

<400> 79
tg          2